# EUROPEAN PATENT APPLICATION

(11) **EP 4 799 565 A1**
(43) Date of publication of application: **02.09.2026**
(21) Application number: 25213885.4
(22) Date of filing: 06.11.2025
(51) Int. Cl.: A61B 5/145

(54) **APPLICATOR AND APPLICATOR ASSEMBLY**

(30) Priority: 07.11.2024 KR 20240157183; 20.08.2025 KR 20250115568
(71) Applicant: i-Sens, Inc., Seoul 06646 (KR)
(72) Inventor: CHOI, Hyun Ho, 06646 Seoul (KR); SEONG, Yul Min, 06646 Seoul (KR); CHAE, Kyung Chul, 06646 Seoul (KR)
(74) Representative: Isarpatent

(57) **Abstract**

According to one aspect of the present invention, an applicator and an applicator assembly capable of stably attaching a wearable unit to skin while inserting a transcutaneous sensor into an accurate subcutaneous position may be provided.

## Description

### BACKGROUND OF THE INVENTION

### Field of the Invention

The present invention relates to an applicator and an applicator assembly, and more specifically, relates to an applicator and an applicator assembly for inserting a transcutaneous sensor that detects biometric information into subcutaneous tissue.

### Description of the Related Art

Chronic diseases such as diabetes mellitus require continuous management. Diabetes mellitus is characterized by having almost no subjective symptoms in an early stage, but when diabetes mellitus progresses, characteristic symptoms such as polydipsia, polyphagia, polyuria, weight loss, general malaise, skin pruritus, cases in which wounds on hands and feet do not heal and last long appear, and the like. When diabetes mellitus progresses further, complications may occur which advance to visual impairment, hypertension, nephropathy, stroke, periodontal disease, muscle cramps and neuralgia, gangrene, and the like. In order to diagnose such diabetes mellitus and to manage such that progression to complications does not occur, systematic blood glucose measurement and treatment need to be performed in parallel.

Patients with diabetes mellitus or persons in whom glucose levels at a reference value or more are detected in blood although progression to diabetes mellitus has not occurred, require continuous and periodic blood glucose measurement for management of diabetes mellitus or prevention of progression to diabetes mellitus.

Currently, a blood sampling method in which blood is collected from a position such as a fingertip and the like, and blood glucose is measured in a unit of a single time constitutes a mainstream of blood glucose measurement methods. However, in the case of the blood sampling-type blood glucose measurement method, due to pain accompanied at the time of blood sampling, inconvenience generated therefrom, and the like, there is a limitation in continuous and periodic blood glucose measurement.

Recently, in order to overcome the limitation of the blood sampling-type blood glucose measurement method, a blood glucose measurement system which measures blood glucose continuously and periodically by inserting a transcutaneous sensor into subcutaneous tissue has been developed and is being used. The blood glucose measurement system using the transcutaneous sensor is generally configured to include a wearable unit provided to be attachable to a human body including the transcutaneous sensor, an applicator which is provided to be coupled to the wearable unit in advance so as to attach the wearable unit to the human body at the same time as inserting the transcutaneous sensor into the subcutaneous tissue, and a receiving unit which processes information received from the wearable unit.

Meanwhile, in terms of hygiene, infection prevention, and the like, the wearable unit is generally provided to be discarded after a single wearing, and the applicator is also generally provided to be discarded after a single firing. That is, when the transcutaneous sensor is not inserted into an accurate subcutaneous position or the wearable unit is not normally attached to the skin, the corresponding wearable unit and the applicator need to each be discarded regardless of their respective usage lifetimes. Therefore, in order to improve the convenience of use and economic efficiency of the blood glucose measurement system using the transcutaneous sensor, there is a need for an applicator which may insert the transcutaneous sensor into an accurate subcutaneous position and may also attach the wearable unit stably to the skin.

### SUMMARY OF THE INVENTION

One aspect of the present invention is to provide an applicator and an applicator assembly capable of stably attaching a wearable unit to skin while inserting a transcutaneous sensor into an accurate subcutaneous position.

The object of the present invention is not limited to the above description. A person with ordinary skill in the art to which the present invention pertains will have no difficulty in readily understanding additional objects of the present invention from the overall contents of the present specification.

The present invention is defined by the independent claims. Further aspects of the present invention are outlined in the dependent claims.

An applicator according to one aspect of the present invention may include: a body housing in which an accommodation portion capable of accommodating a first unit is provided at one end thereof and a movement space connected to the accommodation portion is formed; a first unit carrier moving in the movement space; and a unit support portion provided at one side of the accommodation portion.

The unit support portion may be pressed by one end of the first unit carrier as the first unit carrier moves in a direction becoming closer to the accommodation portion, and may be moved from a restriction position in which at least one end thereof contacts the first unit accommodated in the accommodation portion to a release position in which at least one end thereof moves away from the first unit accommodated in the accommodation portion as the unit support portion is pressed by the first unit carrier.

The first unit may be supported by one end of the unit support portion positioned at the restriction position so that deviation from the accommodation portion may be restricted, and the deviation restriction of the first unit by the unit support portion may be released as the unit support portion moves to the release position.

A second unit, intended to be delivered to the first unit, may be detachably disposed at one end of the first unit carrier, and the first unit carrier may be configured to move together with the second unit from an initial position, in which the second unit is spaced apart from the first unit, toward an insertion position, in which the second unit is delivered to the first unit.

The unit support portion may be pressed by the one end of the first unit carrier which has moved to the insertion position, thereby moving from the restriction position to the release position.

The first unit carrier may move from an initial position spaced apart from the first unit to an insertion position in which the unit support portion is pressed.

The first unit carrier may comprise: a first unit carrier body, at least a portion of which is accommodated in the movement space and which is provided to move in the movement space; an extension arm extending from a side end of the first unit carrier body and formed to extend toward the accommodation portion; and an extension arm pushing portion formed to protrude from a leading end portion of the extension arm adjacent to the accommodation portion.

The extension arm pushing portion may comprise an inclined surface configured to assist the unit support portion in moving to a position away from the accommodation portion.

The unit support portion may comprise: a support hook disposed in a support hook accommodation space formed to penetrate one end of the body housing at one side of the accommodation portion, the support hook comprising a catching portion having a shape protruding toward the accommodation portion and a pushed portion formed to protrude in a direction opposite to the catching portion; and a support hook connection portion extending from an end portion of the body housing forming the support hook accommodation space so as to be connected to the support hook and maintaining the support hook in the support hook accommodation space.

The unit support portion may be moved from the restriction position to the release position as the support hook connection portion is deformed.

The first unit may be provided with a first unit housing groove formed to be recessed in a shape corresponding to the catching portion so that at least one end of the catching portion positioned at the restriction position is inserted and disposed.

The pushed portion may be provided with a pushed portion inclined surface so that the pushed portion slidingly moves, as the pushed portion is pressed in contact with one end of the first unit carrier.

The body housing further may comprise a column disposed in the body housing to partition the movement space, and a carrier slit, in which at least a portion of the extension arm is accommodated, may be formed by incision in an end portion of the column along the first direction.

An applicator assembly, according to one aspect of the present invention, may comprise: a transmission unit intended to be delivered to a detection position on a skin; a body housing in which a transmission unit accommodation portion, capable of accommodating the transmission unit, is provided at one end thereof, and a movement space is formed along a first direction; a sensor unit carrier provided to move in the movement space; and a sensor unit comprising a transcutaneous sensor member capable of detecting biometric information in a subcutaneous tissue of a human body, the sensor unit being disposed to move in the movement space together with the sensor unit carrier, and being configured to move relative to the transmission unit so as to be coupled to the transmission unit.

The applicator assembly may comprise a transmission unit support portion provided at one side of the transmission unit accommodation portion, the transmission unit support portion being pressed by one end of the sensor unit carrier that has moved together with the sensor unit, and moved from a restriction position where at least one end thereof is in contact with the transmission unit accommodated in the transmission unit accommodation portion to a release position where at least one end thereof is spaced apart from the transmission unit.

The transmission unit support portion may switch and move from the restriction position to the release position along a direction intersecting the first direction.

An applicator assembly, according to one aspect of the present invention, may comprise: a body housing in which an accommodation portion, capable of accommodating a transmission unit, is provided at one end thereof, and a movement space connected to the accommodation portion through an opening is formed therein; and a sensor unit carrier provided to move in the movement space, the sensor unit carrier having a sensor unit, intended to be delivered to the transmission unit, separably fixed at one end thereof.

The applicator assembly may further comprise: unit support portions disposed in pairs in a symmetrical shape on both side ends of the opening, the unit support portions having end portions in contact with the transmission unit accommodated in the accommodation portion to restrict deviation of the transmission unit from the accommodation portion.

A spacing width between the paired unit support portions may be wider after completion of the delivery of the sensor unit to the transmission unit than before the delivery of the sensor unit to the transmission unit.

The unit support portion may be integrally formed with the body housing.

The deviation restriction of the transmission unit by the unit support portion may be released as the sensor unit is delivered to the transmission unit.

According to one aspect of the present invention, an applicator and an applicator assembly capable of stably attaching a wearable unit to skin while inserting a transcutaneous sensor into an accurate subcutaneous position may be provided.

An applicator, according to one aspect of the present invention, may comprise: a body housing forming a space capable of accommodating a first unit; and a unit carrier provided to be movable within the space.

The first unit may include a sensor member configured for obtaining a glucose data.

An applicator, according to one aspect of the present invention, comprising: a body housing in which an accommodation portion capable of accommodating a first unit is provided at one end thereof and a movement space connected to the accommodation portion is formed; a first unit carrier configured to move in the movement space.

The housing may include a unit support portion provided on one side of the receiving portion to limit movement of the first unit.

The unit support portion is pressed by one end of the first unit carrier as the first unit carrier moves in a direction becoming closer to the accommodation portion, and is moved from a restriction position in which at least one end thereof contacts the first unit accommodated in the accommodation portion to a release position in which at least one end thereof moves away from the first unit accommodated in the accommodation portion as the unit support portion is pressed by the first unit carrier.

An applicator assembly, according to one aspect of the present invention, may comprise: an applicator configured for ejecting a sensor member; and a cap separably coupled to one end of an applicator.

An applicator, according to one aspect of the present invention, may comprise: a body housing forming an internal space capable of accommodating a first unit having a sensor member; and a handle housing disposed to overlap at least a portion of the body housing.

According to one aspect of the present invention, an applicator assembly may include a sensor member configured to obtain an analyte data.

According to one aspect of the present invention, a sensor member may be a glucose sensor configured to obtain a glucose data.

According to one aspect of the present invention, at least a portion of a sensor member may be electrically coupled with a circuit board.

According to one aspect of the present invention, at least a portion of the sensor member may have a surface facing towards a skin or extend towards the skin.

According to one aspect of the present invention, an applicator and an applicator assembly capable of improving safety of an invasive medical device.

According to one aspect of the present invention, an applicator and an applicator assembly capable of inserting a transcutaneous sensor member into subcutaneous tissue when a predetermined force or more is applied to the applicator.

According to one aspect of the present invention, an applicator and an applicator assembly capable of penetrating the skin at a speed and with a force suitable for a needle insertion.

The effects of the present invention are not limited to the above description, and may include matters that can be reasonably inferred from the following description by a person with ordinary skill in the art to which the present invention pertains.

### BRIEF DESCRIPTION OF THE DRAWINGS

FIG. 1 is a perspective view illustrating an exemplary embodiment of an applicator assembly.
FIG. 2 is a perspective view illustrating an exemplary embodiment of the applicator assembly with a cap removed.
FIG. 3 is a perspective view illustrating an exemplary embodiment of a wearable unit.
FIG. 4 is a conceptual view illustrating an exemplary embodiment of the wearable unit attached to a human body and a remote terminal.
FIG. 5 is a perspective view illustrating an exemplary embodiment of a sensor unit in a state in which a needle is coupled.
FIG. 6 is a perspective view illustrating an exemplary embodiment of a coupling process of the sensor unit and a transmission unit.
FIGS. 7 and 8 are exploded perspective views illustrating an exemplary embodiment of the applicator assembly with the cap removed.
FIG. 9 is a perspective view illustrating an exemplary embodiment of a handle housing.
FIG. 10 is a bottom view illustrating an exemplary embodiment of the handle housing.
FIG. 11 is a perspective view illustrating an exemplary embodiment of a handle housing provided with a guide frame.
FIG. 12 is a bottom view illustrating an exemplary embodiment of a handle housing provided with a guide frame.
FIG. 13 is a cross-sectional view illustrating the handle housing of FIGS. 9 and 10 cut in an A-A' direction.
FIG. 14 is a perspective view and a partially enlarged view illustrating an exemplary embodiment of a body housing.
FIG. 15 is a bottom view illustrating an exemplary embodiment of the body housing.
FIG. 16 is a plan view illustrating an exemplary embodiment of the body housing.
FIG. 17 is a cross-sectional view illustrating the body housing cut along B-B' of FIG. 16.
FIG. 18 is a partial cross-sectional perspective view illustrating the body housing cut along C-C' of FIG. 16.
FIG. 19 is a plan view illustrating an exemplary embodiment of a body housing provided with an auxiliary plate.
FIG. 20 is a partial cross-sectional perspective view of a body housing cut along C-C' of FIG. 19.
FIG. 21 is a bottom view additionally illustrating a position of an auxiliary plate in FIG. 12 to explain a positional relationship between the guide frame and the auxiliary plate.
FIGS. 22 and 23 are perspective views illustrating an exemplary embodiment of a sensor unit carrier.
FIG. 24 is a perspective view exemplarily illustrating a coupling relationship of a needle carrier.
FIG. 25 is a perspective view illustrating an exemplary embodiment of the needle carrier.
FIG. 26 is a front view exemplarily illustrating a coupling relationship of the needle carrier and the sensor unit carrier.
FIG. 27 is an exploded perspective view illustrating an exemplary embodiment of the cap.
FIG. 28 is a cross-sectional view illustrating the cap cut along an E-E' direction in FIG. 27.
FIGS. 29 to 31 are partially enlarged cross-sectional views illustrating an exemplary embodiment of the applicator assembly to which the cap is applied.
FIG. 32 is a partial cross-sectional view illustrating an exemplary embodiment of a state in which the sensor unit carrier is coupled to the body housing during an assembly process of the applicator assembly.
FIGS. 33 and 34 are partial cross-sectional views illustrating an exemplary embodiment of a process of coupling the handle housing to the body housing to which the sensor unit carrier is coupled during an assembly process of the applicator assembly.
FIG. 35 is an enlarged cross-sectional view illustrating an exemplary embodiment of a state in which a leading end portion of a fixing protrusion portion is inserted into a fixing groove during an assembly process of the applicator assembly.
FIG. 36 is an enlarged cross-sectional view illustrating an exemplary embodiment of a state in which the leading end portion of the fixing protrusion portion is discharged from the fixing groove during an assembly process of the applicator assembly.
FIGS. 37 to 39 are partial cross-sectional views sequentially illustrating an exemplary embodiment of a state in which the sensor unit carrier moves from an initial position to an insertion position during an operation process of the applicator assembly.
FIG. 40 is a partial cross-sectional view illustrating an exemplary embodiment of a state in which the needle carrier moves to a retracted position during an operation process of the applicator assembly.
FIG. 41 is a partial cross-sectional view illustrating an exemplary embodiment of a state before a bridge is cut during an operation process of the applicator assembly.
FIG. 42 is a partial cross-sectional view illustrating an exemplary embodiment of a state in which the bridge is cut during an operation process of the applicator assembly.
FIG. 43 is a cross-sectional view and a partial enlarged cross-sectional view for explaining an exemplary embodiment regarding a positional relationship between a first movement restriction portion and a second movement restriction portion in a state before operation of the applicator assembly.
FIGS. 44(a) to 44(c) are sequentially illustrated partial enlarged cross-sectional views for explaining an exemplary embodiment regarding a positional relationship between the first movement restriction portion and the second movement restriction portion during an operation process of the applicator assembly.
FIGS. 45 to 47 are sequentially illustrated cross-sectional views for explaining an exemplary embodiment of pressing and pressing release of a grip arm protrusion portion during an operation process of the applicator assembly.
FIGS. 48 and 49 are sequentially illustrated cross-sectional views for explaining an exemplary embodiment of movement constraint and movement constraint release of a transmission unit by a transmission unit support portion during an operation process of the applicator assembly.

### DETAILED DESCRIPTION OF THE INVENTION

Hereinafter, preferred embodiments of an applicator and an applicator assembly according to one aspect of the present invention will be described in more detail with reference to the accompanying drawings. Embodiments of the present invention may be modified into various forms, and the scope of the present invention should not be construed as being limited to the embodiments described below. These embodiments are provided to more specifically describe the present invention to those skilled in the technical field to which the present invention pertains. Accordingly, a shape of each element illustrated in the drawings may be emphasized or exaggerated for a clearer description.

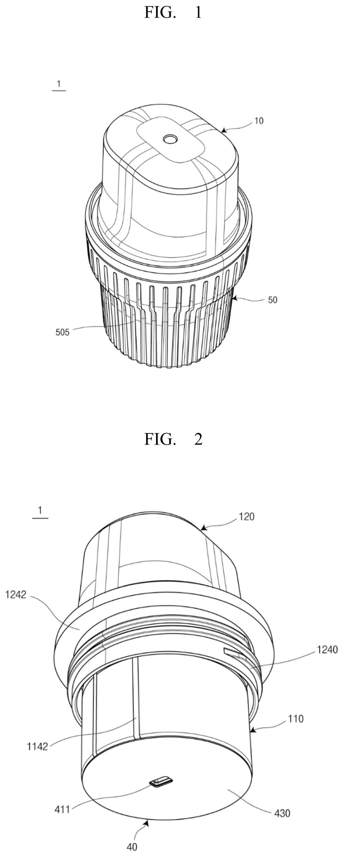

Hereinafter, with reference to FIGS. 1 to 31, the applicator and the applicator assembly will be described in more detail.

### Applicator assembly

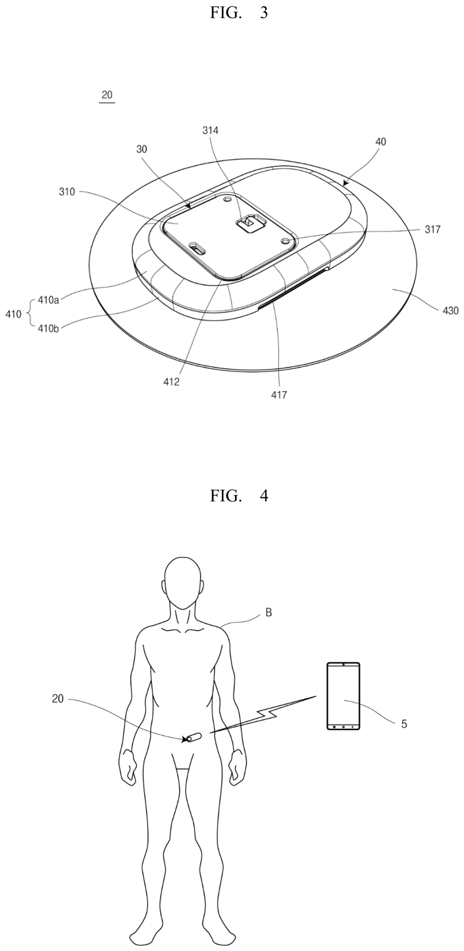

FIG. 1 is a perspective view illustrating an exemplary embodiment of an applicator assembly 1, and FIG. 2 is a perspective view illustrating an exemplary embodiment of the applicator assembly 1 with a cap 50 removed. FIG. 3 is a perspective view illustrating an exemplary embodiment of a wearable unit 20, and FIG. 4 is a conceptual view illustrating an exemplary embodiment of the wearable unit 20 attached to a human body B and a remote terminal 5.

The applicator assembly 1 may include the wearable unit 20 and an applicator 10 that is assembled together with the wearable unit 20 and provided. A user may use the applicator assembly 1 in a state where the wearable unit 20 and the applicator 10 are assembled. The wearable unit 20 and the applicator 10 may be provided to the user in a state assembled during a manufacturing process or a distribution process, and may also be provided to the user in a separated state and then assembled and used by the user before an actual use.

At one end of the applicator 10, the cap 50 may be separably disposed. By the cap 50 separably disposed at one end of the applicator 10, arbitrary firing of the applicator 10 may be prevented, and external contaminants or moisture may be prevented from being introduced into an interior of the applicator assembly 1.

The cap 50 may be detachably coupled to one end of the applicator 10. The cap 50, in a state coupled to the applicator 10, may be disposed so that at least a portion of the applicator 10 and at least a portion of the cap 50 overlap in a horizontal direction.

The wearable unit 20 may include a sensor member 330. The sensor member may be a transcutaneous sensor member. The transcutaneous sensor member may be an incasive sensor or a non-invasive sensor including optical sensor.

The transcutaneous sensor member 330 inserted under the subcutaneous tissue of the human body B and detects biometric information. Although the biometric information detected by the transcutaneous sensor member 330 is various, the preferred biometric information detected by the transcutaneous sensor member 330 may be glucose concentration, ketone, glycated hemoglobin (HbA1c), Fructosamine, 1,5-Anhydroglucitol, other blood-based markers or a combination thereof.

The sensor member 330 may be inserted at least partially into a human body of a user. The sensor member 330 may include an insertion portion, at least a portion of which is inserted into the human body of the user, and a body portion connectable to electronic devices for delivering information acquired from the sensor member 330. The sensor member 330 may further include a connection portion connecting the insertion portion and the body portion. The connection portion may be formed by bending so as to have a predetermined curvature, and the body portion and the insertion portion may be disposed on different planes by the connection portion. The insertion portion may be formed to extend in a direction substantially perpendicular to a surface of the human body to which the wearable unit 20 is attached when the wearable unit 20 is attached to the human body. The body portion may be formed to extend in a direction substantially parallel to a surface of the human body to which the wearable unit 20 is attached when the wearable unit 20 is attached to the human body. A direction in which the insertion portion extends may intersect with a direction in which the body portion extends. The insertion portion, an connection portion, and the body portion may be provided in a plate shape, but shapes of the insertion portion, the connection portion, and the body portion are not necessarily limited to such shapes.

The applicator 10 may be used to deliver the wearable unit 20 onto a detection position on skin such that an end portion of the transcutaneous sensor member 330 included in the wearable unit 20 is inserted into the subcutaneous tissue of the human body B. An adhesive member 430 may be provided at one end of the wearable unit 20. The wearable unit 20 may be maintained at the detection position for a predetermined period of time by the adhesive member 430. The wearable unit 20 may be provided on the applicator 10 in a state where the cap 50 is removed from the applicator assembly 1 so that an adhesive surface of the adhesive member 430 is exposed to the exterior.

The detection position is not limited to a specific position on the human body B, but in terms of convenience in daily life, it may be preferable that the wearable unit 20 is attached on skin of a site of the human body B such as an upper arm, a thigh, an abdomen, or the like.

The wearable unit 20 may be attached to the skin of the human body B, detect biometric information, and wirelessly transmit detected biometric information data to an external terminal 5. A wireless transmission method is not particularly limited, and wireless transmission methods such as Bluetooth (BT), Bluetooth Low Energy (BLE), Near Field Communication (NFC), Radio Frequency Identification (RFID), and the like may be applied. The external terminal 5 is also not particularly limited as long as it is a device capable of receiving and processing data. As a non-limiting example, the external terminal 5 may include a mobile terminal, a dedicated medical device, a PC, a server, and the like. As a non-limiting example, the wearable unit 20 may continuously or periodically detect glucose concentration of the human body B and transmit glucose concentration data to the external terminal 5.

### Wearable unit

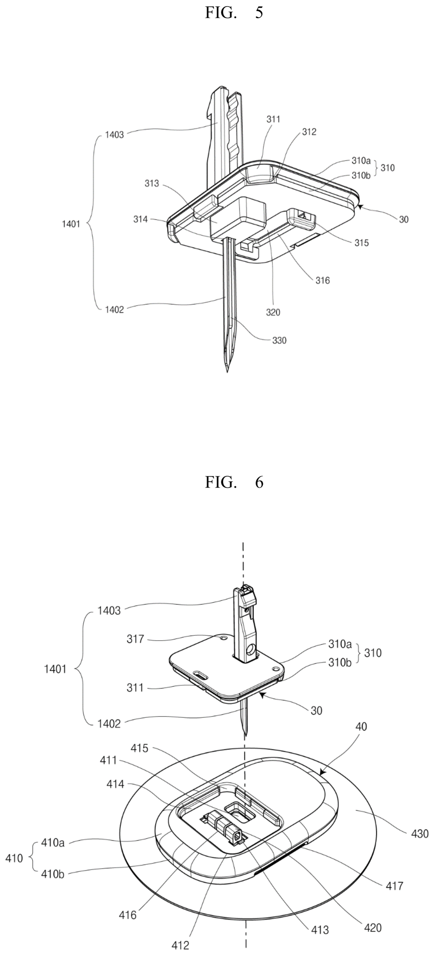

FIG. 5 is a perspective view illustrating an exemplary embodiment of a sensor unit 30 in a state in which a needle 1401 is coupled, and FIG. 6 is a perspective view illustrating an exemplary embodiment of a coupling process of the sensor unit 30 and a transmission unit 40.

The wearable unit 20 may include the sensor unit 30 and the transmission unit 40. The wearable unit 20 may be disposed in an interior of the applicator 10 in an integrated form in which the sensor unit 30 and the transmission unit 40 are coupled. In this case, in a firing process of the applicator 10, the wearable unit 20 in the integrated form in which the sensor unit 30 and the transmission unit 40 are coupled may be attached to skin of the human body B.

Meanwhile, it may include a case in which the sensor unit 30 and the transmission unit 40 are disposed in the applicator 10 in a state separated from each other. In this case, in a process in which the transcutaneous sensor member 330 is inserted into subcutaneous tissue, the sensor unit 30 and the transmission unit 40 may be coupled and delivered to a detection position. It may include all of cases in which the sensor unit 30 and the transmission unit 40 are coupled before the transcutaneous sensor member 330 is inserted into the subcutaneous tissue, or the sensor unit 30 and the transmission unit 40 are coupled simultaneously with insertion of the transcutaneous sensor member 330 into the subcutaneous tissue, or the sensor unit 30 and the transmission unit 40 are coupled after the transcutaneous sensor member 330 is inserted into the subcutaneous tissue.

When the wearable unit 20 is disposed in the interior of the applicator 10 in a state in which the sensor unit 30 and the transmission unit 40 are separated, in an insertion process of the transcutaneous sensor member 330 into the subcutaneous tissue, the sensor unit 30 may relatively move with respect to the transmission unit 40, so that coupling of the sensor unit 30 and the transmission unit 40 may be performed. That is, in a state in which the transmission unit 40 is positioned at the detection position, the sensor unit 30 may move toward the transmission unit 40 so that insertion of the transcutaneous sensor member 330 into the subcutaneous tissue may be performed.

When the sensor unit 30 moves toward the transmission unit 40 so that insertion of the transcutaneous sensor member 330 into the subcutaneous tissue is performed, compared to the wearable unit 20 provided in an integrated form, even when a relatively smaller propulsive force is applied to the transcutaneous sensor member 330, the transcutaneous sensor member 330 may be inserted into an accurate position, and pain and discomfort generated in an insertion process of the transcutaneous sensor member 330 into the subcutaneous tissue may be effectively reduced.

The transmission unit 40 may include a transmission unit housing 410 forming an external shape of the transmission unit 40. A first transmission unit housing 410a and a second transmission unit housing 410b may be coupled to each other to form the transmission unit housing 410. In an interior of the transmission unit housing 410, a battery serving as a power source, an electronic unit for transmitting biometric information data, and the like may be provided. At a coupling portion of the first transmission unit housing 410a and the second transmission unit housing 410b, a sealing portion for preventing external contaminants or moisture from being introduced into the interior of the transmission unit housing 410 may be additionally provided.

An adhesive may be applied to a region where the first transmission unit housing 410a and the second transmission unit housing 410b are coupled, to further reinforce a coupling between the first transmission unit housing 410a and the second transmission unit housing 410b. In this case, the adhesive may function as the sealing portion, or a separate sealing portion other than the adhesive may be provided. The sealing portion may be formed through a separate mechanical configuration such as an O-ring, or may be formed through a chemical configuration such as an adhesive, and the sealing portion may also be formed by using both the mechanical configuration and the chemical configuration.

At one end of the first transmission unit housing 410a, a seating groove 412, in which the sensor unit 30 is accommodated and coupled, may be formed in a shape recessed toward an inner side of the transmission unit housing 410. The seating groove 412 may be formed in a shape recessed toward the inside of the transmission unit housing 410 from one surface of the first transmission unit housing 410a. The seating groove 412 may preferably be formed in a shape corresponding to a sensor unit housing 310 to be described later.

In one region of the first transmission unit housing 410a forming a side surface of the seating groove 412, a side surface protrusion portion 414 having a shape protruding toward a central portion of the seating groove 412 may be provided. The side surface protrusion portion 414 may be divided into a plurality of portions by a dividing portion 415. A sensor unit housing protrusion portion 311, which will be described later, may be disposed on the dividing portion 415. The side surface protrusion portion 414 may be provided to extend along a circumferential direction of the seating groove 412, and may be divided into a plurality of regions by one or more dividing portions 415.

On one surface of the first transmission unit housing 410a forming the seating groove 412, a first connection opening 416 connecting the interior of the transmission unit housing 410 and the exterior may be formed through. A transmission unit connector 420 connected to the electronic unit disposed in the interior of the transmission unit housing 410 may be exposed to the exterior through the first connection opening 416. The transmission unit connector 420 may be disposed to penetrate through the first connection opening 416 so that an end portion of the transmission unit connector 420 protrudes outward of the first connection opening 416. A shape of the transmission unit connector 420 is not limited to a shape illustrated in the drawings. The transmission unit connector 420 is not limited and may be variously modified and applied as long as the transmission unit connector 420 has a shape electrically connectable to a connection terminal intended to be connected to the transmission unit connector 420. It is preferable that the transmission unit connector 420 is provided with a material capable of conducting electricity. It may be more preferable that the transmission unit connector 420 is provided with a material or structure having self-elastic force in terms of contact stability.

At an end portion of the first transmission unit housing 410a forming a boundary with the first connection opening 416, a fastening latch 413 formed to protrude toward an inside of the seating groove 412 may be provided. The fastening latch 413 may be coupled to a fastening ring 315, which will be described later. By being fastened to the fastening ring 315, the fastening latch 413 may assist in maintaining a firm coupled state between the sensor unit 30 and the transmission unit 40.

An insertion hole 411 may be formed in a shape penetrating the first transmission unit housing 410a and the second transmission unit housing 410b from the inside of the seating groove 412. When the transcutaneous sensor member 330 is inserted into subcutaneous tissue, at least part of a needle body 1402 and at least part of the transcutaneous sensor member 330 may pass through the insertion hole 411 and be inserted into the subcutaneous tissue. After an end portion of the transcutaneous sensor member 330 is inserted into the subcutaneous tissue, the needle body 1402 is discharged from skin through the insertion hole 411, and one end of the transcutaneous sensor member 330 may be maintained in a state inserted into the subcutaneous tissue.

At one end of the second transmission unit housing 410b opposite to one end in which the seating groove 412 is formed, the adhesive member 430 may be provided. By the adhesive member 430 attached to skin, the wearable unit 20 may be maintained at a detection position for a predetermined period of time. The adhesive member 430 may include a first adhesive surface adhered to skin and a second adhesive surface adhered to one end of the second transmission unit housing 410b. For protection of the first adhesive surface before attachment to skin, a protective film may be additionally provided on the first adhesive surface adhered to skin. The protective film may be an optional component.

In FIG. 6, the adhesive member 430 provided to have a larger area than one end of the second transmission unit housing 410b is illustrated, but a size or a shape of the adhesive member 430 is not necessarily limited thereto. The adhesive member 430 may be provided to have an area corresponding to one end of the second transmission unit housing 410b, or may be provided to have a smaller/identical area than/to one end of the second transmission unit housing 410b. The adhesive member 430 may be separately provided so that a user attaches it to the transmission unit housing 410 in an insertion process of the transcutaneous sensor member 330 into subcutaneous tissue, but may include a case in which the adhesive member 430 is provided to maintain a state in which the adhesive member 430 is pre-attached to the transmission unit housing 410 disposed in an interior of the applicator assembly 1.

A transmission unit housing groove 417 may be provided on a side end of the transmission unit housing 410. For example, on a side end portion of one end side of the second transmission unit housing 410b facing the adhesive member 430, a transmission unit housing groove 417 formed to be recessed toward the inside of the transmission unit housing 410 may be provided. The transmission unit housing groove 417 may be formed to be recessed in a symmetrical shape at both side end portions facing each other of the first transmission unit housing 410a. Meanwhile, the transmission unit housing groove 417 may be provided in a plurality of pairs, and these transmission unit housing grooves 417 may be spaced apart from each other along a circumferential direction of the second transmission unit housing 410b and formed at symmetrical positions with respect to the second transmission unit housing 410b. An end portion of a catching portion 1164 provided in a transmission unit support portion 1160 to be described later may be disposed in an interior of the transmission unit housing groove 417, and in this case, the transmission unit 40 may be effectively prevented from being arbitrarily deviated from the applicator 10.

The transmission unit housing groove 417 may be formed only in the second transmission unit housing 410b, or may be formed in the first transmission unit housing 410a and the second transmission unit housing 410b to form an integrated groove. When the transmission unit housing groove 417 is formed in both the first transmission unit housing 410a and the second transmission unit housing 410b, the transmission unit housing groove 417 may be visible to a user in a situation where the user looks down on the transmission unit housing 410 (or in a situation where the user looks at the first transmission unit housing 410a in a state where the transmission unit housing 410 is attached to the detection position of the user). Meanwhile, when the transmission unit housing groove 417 is formed only in the second transmission unit housing 410b, the transmission unit housing groove 417 may not be visible to the user in a situation where the user looks down on the transmission unit housing 410 (or in a situation where the user looks at the first transmission unit housing 410a in a state where the transmission unit housing 410 is attached to the detection position of the user).

The sensor unit 30 may include a sensor unit housing 310 forming an external shape of the sensor unit 30, and a transcutaneous sensor member 330 disposed in the interior of the sensor unit housing 310 and provided such that one end thereof extend to an exterior of the sensor unit housing 310. When the wearable unit 20 is attached to the human body B, at least one end of the transcutaneous sensor member 330 extended to the exterior of the sensor unit housing 310 may be maintained in a state inserted into a subcutaneous tissue. Biometric information may be detected by the transcutaneous sensor member 330 inserted subcutaneously through the skin.

The sensor unit housing 310 may be formed by coupling a first sensor unit housing 310a and a second sensor unit housing 310b to each other. At a coupling portion of the first sensor unit housing 310a and the second sensor unit housing 310b, a sealing portion for preventing external contaminants or moisture from being introduced into the interior of the sensor unit housing 310 may be additionally provided.

At least one adhesive member may be disposed between the sensor unit housing 310 and the sensor member 330. The adhesive member may be provided to have an adhesive force on both surfaces and may further reinforce a coupling between the sensor member 330 and the sensor unit housing 310. For example, a double-sided tape may be disposed on at least one surface of an upper portion or a lower portion of a body portion of the sensor member 330, and the sensor member 330 and the sensor unit housing 310 may maintain a firm coupling force with each other by the double-sided tape disposed between the body portion of the sensor member 330 and the sensor unit housing 310. Meanwhile, although a case where the double-sided tape is used as the adhesive member has been exemplarily described, the adhesive member is not limited to the double-sided tape and may be variously modified and used as long as it is a means capable of improving a coupling force between the sensor member 330 and the sensor unit housing 310.

At a side end portion of the second sensor unit housing 310b, a plurality of concave portions 312 having shapes recessed from a side surface of the second sensor unit housing 310b may be formed spaced apart at predetermined intervals along a circumferential direction of the second sensor unit housing 310b, and on one surface of the first sensor unit housing 310a facing the second sensor unit housing 310b, a sensor unit housing protrusion portion 311 formed to protrude at positions corresponding to the plurality of concave portions 312 may be provided.

The sensor unit housing protrusion portion 311 and the concave portion 312 may be provided to have a shape in which the sensor unit housing protrusion portion 311 is in close contact with the concave portion 312 or the sensor unit housing protrusion portion 311 is capable of pressing the concave portion 312 in a state in which the first sensor unit housing 310a and the second sensor unit housing 310b are coupled. Since the sensor unit housing protrusion portion 311 is maintained in a state of being in close contact with or pressing the concave portion 312 in a state in which the first sensor unit housing 310a and the second sensor unit housing 310b are coupled to each other, the first sensor unit housing 310a and the second sensor unit housing 310b may be maintained in a more firmly coupled state. The sensor unit housing protrusion portion 311 and the concave portion 312 may be coupled to each other by a forced fitting method.

Meanwhile, the side surface protrusion portion 414 formed in the transmission unit housing 410 may be provided to have a shape closely contacting a side end of the second sensor unit housing 310b or pressing the side end of the second sensor unit housing 310b in a state where the sensor unit 30 and the transmission unit 40 are coupled. By close contact of the side surface protrusion portion 414 and the side end of the second sensor unit housing 310b, or by pressing of the second sensor unit housing 310b by the side surface protrusion portion 414, the sensor unit 30 and the transmission unit 40 may maintain a more firmly fixed state.

The sensor unit housing protrusion portion 311 may be formed at a position corresponding to the dividing portion 415 formed at a side end of the seating groove 412. During a process in which the sensor unit 30 and the transmission unit 40 are coupled, the dividing portion 415 may guide an entry position of the sensor unit housing protrusion portion 311, and the sensor unit 30 may be stably introduced into the seating groove 412. When maintaining a state in which the sensor unit 30 and the transmission unit 40 are coupled, the sensor unit housing protrusion portion 311 may be disposed in an interior of the dividing portion 415 formed at a side end of the seating groove 412. The sensor unit housing protrusion portion 311 may be closely contacted with one surface of the first transmission unit housing 410a forming the dividing portion 415, or one surface of the first transmission unit housing 410a forming the dividing portion 415 may press the sensor unit housing protrusion portion 311.

At one end of the second sensor unit housing 310b disposed to face the seating groove 412 during coupling of the sensor unit 30 and the transmission unit 40, a second connection opening 316 connecting an interior of the sensor unit housing 310 and the exterior may be formed through. A sensor unit connector 320 connected to the transcutaneous sensor member 330 may be exposed to the exterior through the second connection opening 316. When the sensor unit 30 and the transmission unit 40 are coupled, the sensor unit connector 320 and the transmission unit connector 420 may be electrically contacted with each other. Biometric information data detected by the transcutaneous sensor member 330 may be delivered to an electronic unit provided in the transmission unit 40 through the sensor unit connector 320 and the transmission unit connector 420.

The sensor unit connector 320 may be formed on one surface of the transcutaneous sensor member 330 corresponding to the second connection opening 316, with a size and shape corresponding to a size and shape of the second connection opening 316. The sensor unit connector 320 may be formed on one surface of the transcutaneous sensor member 330 corresponding to the second connection opening 316 with a smaller size than the second connection opening 316, or may be formed on one surface of the transcutaneous sensor member 330 corresponding to the second connection opening 316 with a larger size than the second connection opening 316. In terms of electrical contact stability, the sensor unit connector 320 may preferably be formed to have a larger area than one surface of the transmission unit connector 420 contacting the sensor unit connector 320. The transmission unit connector 420 may be provided in a shape divided into a plurality, and the transmission unit connectors 420 divided into a plurality may be disposed to be spaced apart from each other. Meanwhile, the transmission unit connector 420 may include a case where a divided region is formed only in a one end portion side region contacting the sensor unit connector 320. The sensor unit connector 320 may be formed on one surface of the transcutaneous sensor member 330 in a form divided into a plurality, respectively corresponding to the transmission unit connectors 420 divided into a plurality, and may also be formed integrally on one surface of the transcutaneous sensor member 330 so as to correspond to all of the transmission unit connectors 420 divided into a plurality.

The transmission unit connector 420 may include a body portion and a coating layer formed on a surface of the body portion. The coating layer may be formed by plating. The coating layer formed on the transmission unit connector 420 may be formed as a single layer, or may also be formed as a plurality of layers.

The body portion of the transmission unit connector 420 may be formed of an elastic material and may be pressed and compressed in a state of being in contact with the sensor unit connector 320. That is, a vertical height (or a first direction height) of the transmission unit connector 420 when the sensor unit 30 and the transmission unit 40 are separated may be higher than a vertical height (or a first direction height) of the transmission unit connector 420 when the sensor unit 30 and the transmission unit 40 are coupled.

At one end of the second sensor unit housing 310b facing the transmission unit 40, a boss 313 formed to protrude in a shape corresponding to the insertion hole 411 may be provided. A through-hole 314 communicating an interior and an exterior of the sensor unit housing 310 may be formed to penetrate the boss 313.

One end of the transcutaneous sensor member 330, intended to be inserted into the subcutaneous tissue, may extend from the interior of the sensor unit housing 310 to the exterior of the sensor unit housing 310 through the through-hole 314 and may be disposed. The through-hole 314 may be formed to extend through not only the second sensor unit housing 310b but also the first sensor unit housing 310a. The needle body 1402 may be disposed to pass through the through-hole 314 in a state in which the sensor unit 30 and the needle 1401 are coupled. In this case, one end of the transcutaneous sensor member 330 extending to the exterior of the sensor unit housing 310 may be disposed in the exterior of the sensor unit housing 310 in a state accommodated in an interior of the needle body 1402.

When the sensor unit 30 and the transmission unit 40 are coupled, a circumferential direction side surface of the boss 313 and an inner surface of the transmission unit housing 410 forming the insertion hole 411 may be closely contacted or may be disposed in a closely adjacent position with each other. Accordingly, not only may one end of the transcutaneous sensor member 330 be inserted into an accurate position, but also the sensor unit 30 and the transmission unit 40 may be maintained in a more firmly coupled state.

Meanwhile, in a state in which the sensor unit 30 and the transmission unit 40 are coupled to each other, the circumferential direction side surface of the boss 313 and the inner surface of the transmission unit housing 410 forming the insertion hole 411 may be maintained in a state closely contacted or closely disposed with each other, and accordingly, in a state in which the wearable unit 20 is attached to the human body B, external contaminants or moisture may be effectively prevented from being introduced toward the sensor unit connector 320 and the transmission unit connector 420.

At an end portion of the second sensor unit housing 310b forming a boundary with the second connection opening 316, the fastening ring 315 formed to protrude along a direction parallel to a protruding direction of the transcutaneous sensor member 330 may be provided. The fastening ring 315 may be provided in a pair in a symmetrical shape on both sides of the second connection opening 316, and the fastening latch 413 may also be provided in pairs in a symmetrical shape at positions corresponding to the fastening ring 315. In a coupling process of the sensor unit 30 and the transmission unit 40, the fastening latch 413 may be fixed to the fastening ring 315, and by mutual coupling of the fastening latch 413 and the fastening ring 315, the sensor unit 30 and the transmission unit 40 may be maintained in a firmly coupled state.

In the above, an example is described in which the fastening latch 413 is provided in the first transmission unit housing 410a, and the fastening ring 315 is provided in the second sensor unit housing 310b, but it may include a case in which the fastening latch is provided in the second sensor unit housing 310b, and the fastening ring 315 is provided in the first transmission unit housing 410a. Meanwhile, fastening means of the sensor unit 30 and the transmission unit 40 are not limited to a latch and a ring. In an insertion process of the transcutaneous sensor member 330 into subcutaneous tissue, as long as it is a means capable of preventing the sensor unit 30 from being separated from the transmission unit 40 after coupling of the sensor unit 30 and the transmission unit 40, while not interfering with movement of the sensor unit 30 toward the transmission unit 40, it may be variously modified and applied as the fastening means.

On one surface of the first sensor unit housing 310a, a fixing groove 317 may be formed to be recessed, and a leading end portion of a fixing protrusion 1317 formed in a sensor unit carrier 130 to be described later may be inserted into the fixing groove 317 and be disposed. In an insertion process of the transcutaneous sensor member 330 into subcutaneous tissue, the sensor unit 30 may move toward the transmission unit 40 together with the sensor unit carrier 130, and during movement of the sensor unit 30, a leading end portion of the fixing protrusion 1317 may be maintained in a state inserted into the fixing groove 317. The sensor unit 30 may be stably supported by the sensor unit carrier 130 and may be moved. After insertion of the transcutaneous sensor member 330 into the subcutaneous tissue, in a process in which a user removes the applicator 10 from skin, the fixing protrusion 1317 may be discharged from the fixing groove 317, and thereby, a fixing relationship between the sensor unit carrier 130 and the sensor unit 30 may be released.

An adhesive member may be additionally provided between the sensor unit 30 and the transmission unit 40. In a state before coupling of the sensor unit 30 and the transmission unit 40, the adhesive member may be disposed on one surface of the second sensor unit housing 310b facing the seating groove 412, or the adhesive member may be disposed in an interior of the seating groove 412 facing the second sensor unit housing 310b, or the adhesive member may be disposed on both one surface of the second sensor unit housing 310b and the interior of the seating groove 412. By the adhesive member disposed between the sensor unit 30 and the transmission unit 40, the sensor unit 30 and the transmission unit 40 may maintain a firm coupled state, and in a state in which the wearable unit 20 is attached to a detection position of a user, deviation of the sensor unit 30 from the transmission unit 40 may be prevented.

In a state in which the sensor unit 30 and the transmission unit 40 are coupled, the first sensor unit housing 310a, the first transmission unit housing 410a, and the second transmission unit housing 410b may form an appearance of the wearable unit 20.

In a state in which the sensor unit 30 and the transmission unit 40 are coupled, an exterior surface of the first sensor unit housing 310a and an exterior surface of the first transmission unit housing 410a may form one (a single) surface, but may form a discontinuous surface. In a state in which the sensor unit 30 and the transmission unit 40 are coupled, an exterior surface of the first sensor unit housing 310a and an exterior surface of the first transmission unit housing 410a may be disposed at substantially corresponding positions, or the exterior surface of the first sensor unit housing 310a may be disposed at a position more recessed than the exterior surface of the first transmission unit housing 410a. Since the exterior surface of the first sensor unit housing 310a is positioned corresponding to or recessed relative to the exterior surface of the first transmission unit housing 410a, it is possible to mitigate and prevent unintended external impacts from being directly delivered to the sensor unit 30 in a state in which the wearable unit 20 is attached on a detection position of a user, and to effectively prevent damage or deviation of the sensor unit 30 caused by the external impacts.

### Applicator

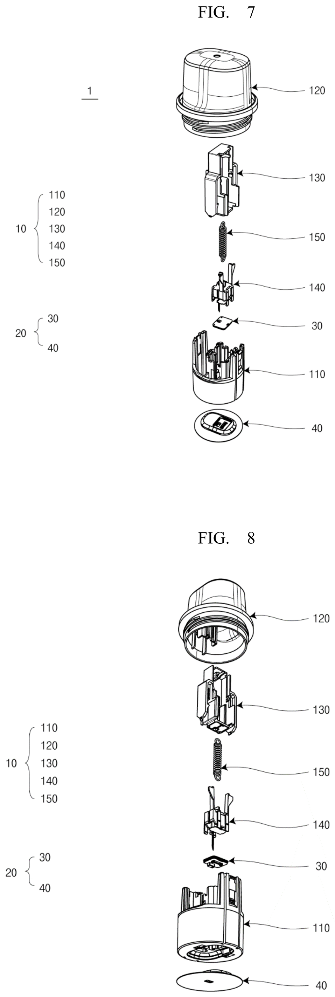

FIGS. 7 and 8 are exploded perspective views illustrating an exemplary embodiment of the applicator assembly 1 with the cap 50 removed. Hereinafter, for convenience of description, a direction generally parallel with an insertion direction of the transcutaneous sensor member 330 is defined as a first direction, and all directions generally perpendicular to the first direction are defined as a second direction, and a specific configuration of the applicator 10 and the applicator assembly 1 is described.

The applicator 10 may be provided to deliver the wearable unit 20 to a detection position on skin. The applicator 10 may include a body housing 110 to which the transmission unit 40 is detachably coupled at one end, a handle housing 120 disposed to relatively move with respect to the body housing 110 along the first direction during insertion of the transcutaneous sensor member 330 into subcutaneous tissue, a sensor unit carrier 130 to which the sensor unit 30 is detachably coupled at one end and which is disposed to move together with the handle housing 120 along the first direction, a needle carrier 140 provided with the needle body 1402 for insertion of the transcutaneous sensor member 330 into subcutaneous tissue and separably and fixedly disposed to the sensor unit carrier 130, and an elastic member 150 having one end and the other end connected to the sensor unit carrier 130 and the needle carrier 140, respectively, and providing driving force such that the needle body 1402 inserted into the subcutaneous tissue is discharged from the human body B.

The applicator assembly 1 may include, in addition to the above-described applicator 10, the transmission unit 40 separably fixed and disposed at one end of the body housing 110, and the sensor unit 30 separably disposed at one end of the sensor unit carrier 130. The applicator assembly 1 may further include the cap 50 detachably coupled to the handle housing 120 to block exterior exposure of the body housing 110 in which the transmission unit 40 is disposed.

### Handle housing

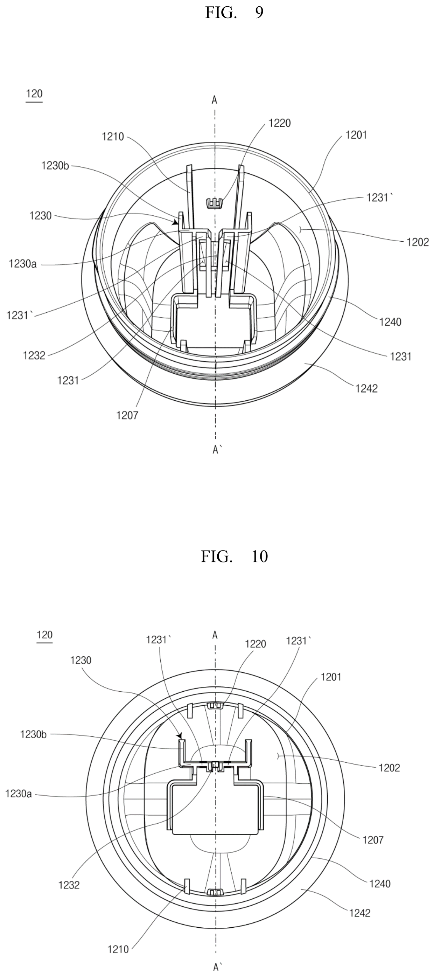

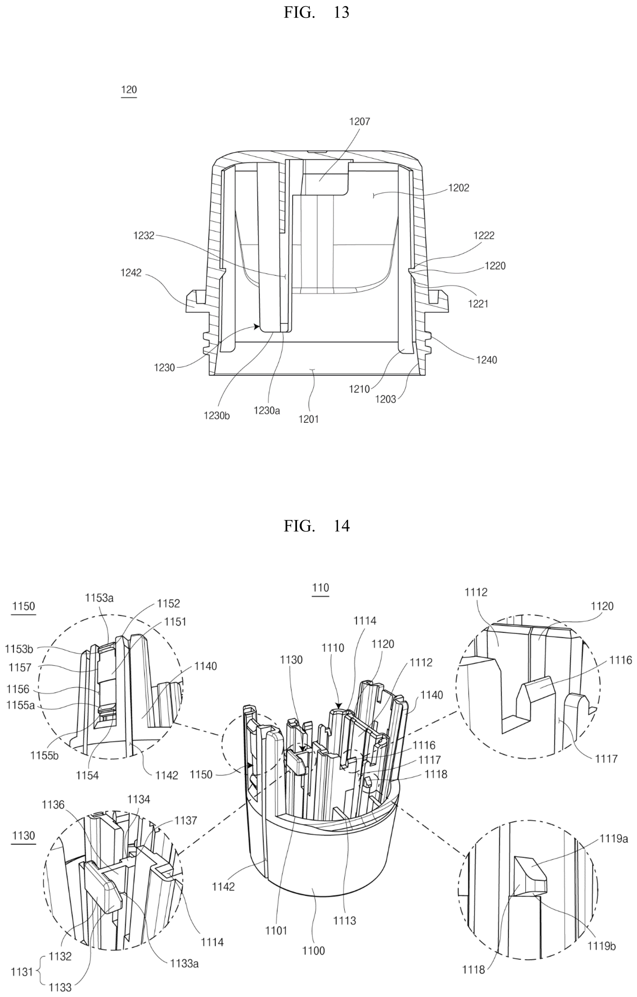

FIGS. 9 and 10 are a perspective view and a bottom view illustrating an exemplary embodiment of the handle housing 120, and FIG. 13 is a cross-sectional view illustrating the handle housing 120 of FIGS. 9 and 10 cut in an A-A' direction.

The handle housing 120 may form an external shape of the applicator 10 together with the body housing 110. The handle housing 120 may be gripped or pressed by a user in an insertion process of the transcutaneous sensor member 330 into subcutaneous tissue. Although FIG. 9, FIG. 10, and FIG. 13 illustrate a cup-shaped handle housing 120, a shape of the handle housing 120 is not necessarily limited to the cup shape. As long as it is a shape capable of achieving functions described below, the handle housing 120 may be modified and applied in various shapes. However, at one end of the handle housing 120, the cap 50 may be detachably coupled. The cap may be detachably coupled by screw-coupling, and therefore, it may be more preferable that one end of the handle housing 120 in which a screw thread for the screw-coupling is formed is provided to have a cylindrical structure.

In an interior of the handle housing 120, a first interior space 1202 may be formed, and the first interior space 1202 may be connected to the exterior by a first opening 1201 formed at one end of the handle housing 120. A push arm 1230 may be formed to extend along the first direction from an inner one surface of the handle housing 120 facing the first opening 1201.

The push arm 1230 may be formed to extend along the first direction from a position eccentric to one side with respect to a central portion of an inner one surface of the handle housing 120 facing the first opening 1201, and may be formed to extend along the first direction at a position spaced apart from an inner side surface of the handle housing 120. The push arm 1230 may be formed to extend to a region not penetrating the first opening 1201. The push arm 1230 may include a main push arm plate 1230a disposed substantially parallel to an adjacent carrier fixing fence 1207, and a pair of extension push arm plates 1230b provided to extend respectively from both side ends of the main push arm plate 1230a. The pair of extension push arm plates 1230b may be disposed in a direction substantially perpendicular to the main push arm plate 1230a, and the pair of extension push arm plates 1230b may be disposed substantially parallel to each other. The push arm 1230 may be provided to interact with a fixing portion 1130 to be described later. In an assembly process of the applicator 10, by pressing and moving the fixing portion 1130 by the push arm 1230, a temporary movement restriction of the sensor unit carrier 130 by the fixing portion 1130 may be released. The push arm 1230 may be means for pressing and moving the fixing portion 1130 in the assembly process of the applicator 10.

A push arm slit 1232 may be formed to extend from a leading end portion of the push arm 1230 in a direction opposite to a protruding direction of the push arm 1230 in a shape dividing an end portion of the push arm 1230. An extension portion 1136 provided in the fixing portion 1130 to be described later may be introduced into the push arm slit 1232, so that during an assembly process of the applicator 10 or in an insertion process of the transcutaneous sensor member 330 into subcutaneous tissue, movement interference of the extension portion 1136 with respect to the push arm 1230 may be excluded. Although FIG. 9, FIG. 10, and FIG. 13 illustrate the push arm 1230 formed symmetrically based on the push arm slit 1232, a shape of the push arm 1230 is not necessarily limited thereto. As long as it is a structure capable of pressing and moving the fixing portion 1130 in an assembly process of the applicator 10, the push arm 1230 may be variously modified and applied.

On one side of the push arm slit 1232, a wing portion accommodation hole 1231 penetrating the push arm 1230 in a thickness direction may be formed in a shape extending along the first direction. Meanwhile, the wing portion accommodation hole 1231 may not completely penetrate the push arm 1230 in the thickness direction, but may be formed in a shape recessed from one surface of the push arm 1230, which comes into contact with a pressing movement wing portion 1133 as the handle housing 120 moves in the first direction. The wing portion accommodation hole 1231 may be provided in a shape and position corresponding to the pressing movement wing portion 1133. When the pressing movement wing portions 1133 are provided in pairs, the wing portion accommodation holes 1231 may be formed symmetrically based on the push arm slit 1232.

In a region of an end portion side of the push arm 1230, a barrier 1231' having a shape closing the wing portion accommodation hole 1231 may be provided. That is, the push arm slit 1232 may be provided in a shape extending to a leading end portion of the push arm 1230, while the wing portion accommodation hole 1231 may be provided in a shape not extending to the leading end portion of the push arm 1230 due to the barrier 1231'. During movement of the handle housing 120 along the first direction, at least one end of the pressing movement wing portion 1133 may be introduced into an interior of the wing portion accommodation hole 1231. The one end of the pressing movement wing portion 1133 introduced into the wing portion accommodation hole 1231 may come into contact with the barrier 1231', thereby restricting the push arm 1230 from moving in a direction opposite to the first direction.

The wing portion accommodation hole 1231, the barrier 1231', and the push arm slit 1232 may be formed in the main push arm plate 1230a. The push arm slit 1232 may be formed at a central portion side of the main push arm plate 1230a, and the wing portion accommodation holes 1231 and the barriers 1231' may be respectively provided in pairs in an outer region of the push arm slit 1232.

On an inner one surface of the handle housing 120 facing the first opening 1201, the carrier fixing fence 1207 disposed at a position adjacent to the push arm 1230 may be provided. The carrier fixing fence 1207 may be disposed upright in a shape corresponding to an entire or partial circumferential portion at one end portion side of the sensor unit carrier 130. The carrier fixing fence 1207 may be provided in a shape surrounding a central portion of an inner one surface of the handle housing 120. The carrier fixing fence 1207 may be provided to have a width larger than the push arm 1230, and may be formed in a shape protruding from the inner one surface of the handle housing 120 with a protrusion height lower than the push arm 1230. After assembly of the applicator 10, the sensor unit carrier 130 may be disposed at a position in which at least one surface of the sensor unit carrier 130 is adjacent to an inner surface of the carrier fixing fence 1207 or in close contact with the inner surface of the carrier fixing fence 1207. In this case, the sensor unit carrier 130 may be disposed at a position in which one end portion of the sensor unit carrier 130 is spaced apart at a predetermined interval from an inner one surface of the handle housing 120 facing the first opening 1201, or at a position in which one end portion of the sensor unit carrier 130 is in contact with the inner one surface of the handle housing 120 facing the first opening 1201.

When a user presses the handle housing 120 in the first direction for insertion of the transcutaneous sensor member 330 into subcutaneous tissue, the sensor unit carrier 130 may be switched to a state in which one end portion of the sensor unit carrier 130 is in contact with the inner one surface of the handle housing 120 facing the first opening 1201, or may maintain a state in which it is in contact with the inner one surface of the handle housing 120 facing the first opening 1201, and may move together with the handle housing 120 along the first direction. In this case, the sensor unit carrier 130 may move in the first direction while maintaining a state in which at least one surface of the sensor unit carrier 130 is firmly in close contact with the inner one surface of the carrier fixing fence 1207, and after completion of insertion of the transcutaneous sensor member 330 into the subcutaneous tissue, it is possible to effectively prevent the handle housing 120 from returning to an initial position.

Meanwhile, the sensor unit carrier 130 may move together with the handle housing 120 in the first direction while maintaining a state spaced apart at a predetermined interval from the inner one surface of the handle housing 120 facing the first opening 1201, and during an insertion process of the transcutaneous sensor member 330 into the subcutaneous tissue or after completion of the insertion of the transcutaneous sensor member 330 into the subcutaneous tissue, one end portion of the sensor unit carrier 130 may be switched to a state in which it is in contact with the inner one surface of the handle housing 120 facing the first opening 1201. In this case, at least one surface of the sensor unit carrier 130 may be more firmly in close contact with the inner one surface of the carrier fixing fence 1207 during the insertion process of the transcutaneous sensor member 330 into the subcutaneous tissue or after completion of the insertion of the transcutaneous sensor member 330 into the subcutaneous tissue, and as one end of the sensor unit carrier 130 engages with the carrier fixing fence 1207, it is possible to effectively prevent the handle housing 120 from returning to the initial position.

On an inner side surface of the handle housing 120 forming the first interior space 1202, a guide protrusion 1210 extending along the first direction in a shape protruding toward the first interior space 1202 may be provided. A leading end portion of the guide protrusion 1210 may be disposed in an interior of the handle housing guide groove 1142 formed to be recessed in an outer side surface of the body housing 110 to be described later. Through interaction of the guide protrusion 1210 and the handle housing guide groove 1142, the movement direction of the handle housing 120 moving in the first direction may be guided. The guide protrusion 1210 may be provided in pairs in a symmetrical shape on an inner side surface of the handle housing 120, and adjacent guide protrusions 1210 may be disposed spaced apart from each other by a distance smaller than a width of the push arm 1230 or a width of the carrier fixing fence 1207.

The handle housing guide groove 1142 may also be formed to be recessed in a shape corresponding to the guide protrusion 1210 at a position corresponding to the guide protrusion 1210. Meanwhile, although a case in which the guide protrusion 1210 is formed on the handle housing 120 and the handle housing guide groove 1142 is formed on the body housing 110 has been exemplarily described, cases in which a guide groove is formed on the handle housing and a guide protrusion is formed on the body housing may be included.

The first movement restriction portion 1220 may be formed to protrude toward a central portion of the first interior space 1202 from the inner side surface of the handle housing 120 forming the first interior space 1202 of the handle housing 120. The first movement restriction portion 1220 may be provided at a position between a plurality of guide protrusions 1210 disposed adjacent to one side of the handle housing 120, or may be provided at a position facing the push arm slit 1232. The first movement restriction portion 1220 may be provided in pairs symmetrically on an inner side surface of the handle housing 120. The first movement restriction portion 1220 provided symmetrically and the push arm slit 1232 may be disposed on the same extension line A-A'. The first movement restriction portion 1220 may be provided to have a width larger than the push arm slit 1232.

The first movement restriction portion 1220 may interact with the second movement restriction portion 1150 provided in the body housing 110 to be described later. By the interaction between the first movement restriction portion 1220 and the second movement restriction portion 1150, it is possible to restrict the handle housing 120 coupled to the body housing 110 from being arbitrarily deviated from the body housing 110, or to restrict the handle housing 120 from moving in a direction opposite to the first direction after insertion of the transcutaneous sensor member 330 into subcutaneous tissue.

The first movement restriction portion 1220 may be provided as a latch structure having a wedge-shaped cross-section provided with an inclined surface 1221 and a support surface 1222, but a shape of the first movement restriction portion 1220 is not necessarily limited thereto. The first movement restriction portion 1220 may be applied without limitation as long as it is a shape capable of restricting the handle housing 120 from being arbitrarily deviated from the body housing 110 through interaction with the second movement restriction portion 1150, or restricting the handle housing 120 from moving in a direction opposite to the first direction after subcutaneous insertion of the transcutaneous sensor member 330. The first movement restriction portion 1220 may be means for restricting the handle housing 120 from being arbitrarily deviated from the body housing 110, or means for restricting the handle housing 120 from moving in a direction opposite to the first direction after insertion of the transcutaneous sensor member 330 into the subcutaneous tissue through interaction with the second movement restriction portion 1150.

On an outer surface of one end portion side of the handle housing 120 where the first opening 1201 is formed, a screw threaded portion 1240 for screw-coupling with the cap 50 may be provided. The screw threaded portion 1240 formed in the handle housing 120 and the screw threaded portion 540 formed in the cap 50 to be described later may be screw-coupled so that the handle housing 120 and the cap 50 may be coupled to each other in a detachable manner.

On an outer surface of the handle housing 120, a locking protrusion portion 1242 protruding toward the outside from the outer surface of the handle housing 120 and extending along a circumferential direction of the handle housing 120 may be provided. The locking protrusion portion 1242 may be provided at a position adjacent to a region where the screw threaded portion 1240 is formed. Since the locking protrusion portion 1242 may be provided in a structure capable of contacting a leading end portion of the cap 50, during screw-coupling between the handle housing 120 and the cap 50, it is possible to prevent excessive tightening between the screw threaded portion 1240 of the handle housing 120 and the screw threaded portion 540 of the cap 50. Meanwhile, in a state in which the handle housing 120 and the cap 50 are mutually coupled, the locking protrusion portion 1242 and an end portion of the cap 50 may be provided to be in close contact, and by such close contact structure, it is possible to effectively prevent external contaminants or moisture from being introduced into an interior of the applicator assembly 1.

As illustrated in FIG. 13, the first movement restriction portion 1220, the locking protrusion portion 1242, and the screw threaded portion 1240 may be sequentially disposed on the handle housing 120 along the first direction, the first movement restriction portion 1220 may be disposed inside the handle housing 120, and the locking protrusion portion 1242 and the screw threaded portion 1240 may be disposed outside the handle housing 120.

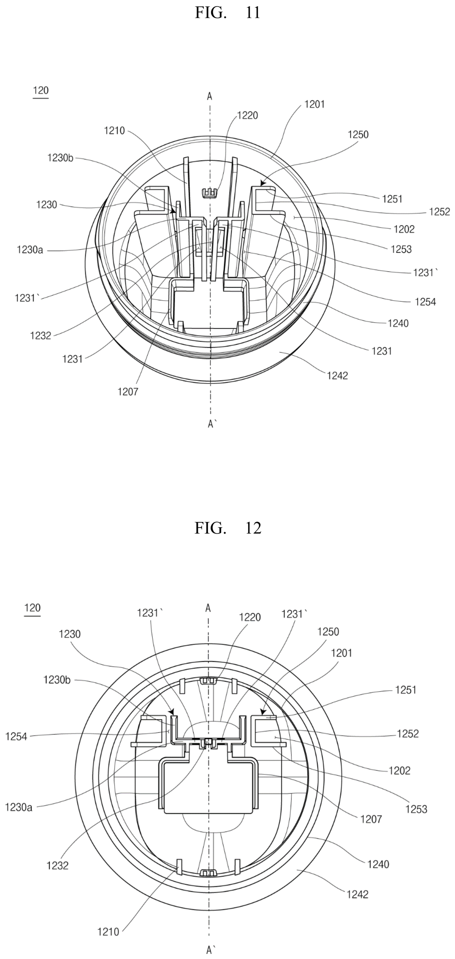

FIG. 11 is a perspective view illustrating an exemplary embodiment of the handle housing 120 provided with a guide frame 1250, and FIG. 12 is a bottom view illustrating an exemplary embodiment of the handle housing 120 provided with the guide frame 1250.

The handle housing 120 may further include the guide frame 1250 disposed at both side ends of the push arm 1230. The guide frame 1250 may be disposed in pairs and may be disposed at intervals from the push arm 1230 so as to be in a symmetrical shape with respect to the push arm 1230. The guide frame 1250 may include a guide plate 1252 disposed to extend along a direction parallel to the extension push arm plate 1230b at a position spaced apart from the extension push arm plate 1230b by a predetermined interval, a first support plate 1251 extending from one end of the guide plate 1252 located relatively adjacent to an inner side wall of the handle housing 120, and a second support plate 1253 extending from the other end of the guide plate 1252 located at a position relatively distant from the inner side wall of the handle housing 120. The guide plate 1252, the first support plate 1251, and the second support plate 1253 may all be formed to extend along the first direction from an inner one surface of the handle housing 120 facing the first opening 1201. The guide plate 1252, the first support plate 1251, and the second support plate 1253 may be formed to extend to a height corresponding to the push arm 1230, or may be formed to extend to a region closer to the first opening 1201 than a leading end of the push arm 1230. The guide plate 1252 may be provided to have a width corresponding to the extension push arm plate 1230b. The guide space 1254 extending along the first direction with a predetermined width may be formed between the guide plate 1252 and the extension push arm plate 1230b. In a coupling process of the body housing 110 and the handle housing 120, at least one end of a first auxiliary plate 1171 to be described later may be introduced into the guide space 1254 and disposed in an interior of the guide space 1254.

The first support plate 1251 and the second support plate 1253 may be disposed in a direction substantially perpendicular to the guide plate 1252, and accordingly, the first support plate 1251 and the second support plate 1253 may be disposed substantially parallel to each other. The second support plate 1253 may be provided to have a width relatively larger than the first support plate 1251. The guide plate 1252 may be more firmly supported by the first support plate 1251 and the second support plate 1253 provided at one end and the other end of the guide plate 1252. End portions of the first support plate 1251 and the second support plate 1253 may be formed in a shape corresponding to a shape of the transmission unit accommodation portion 1104.

### Body housing

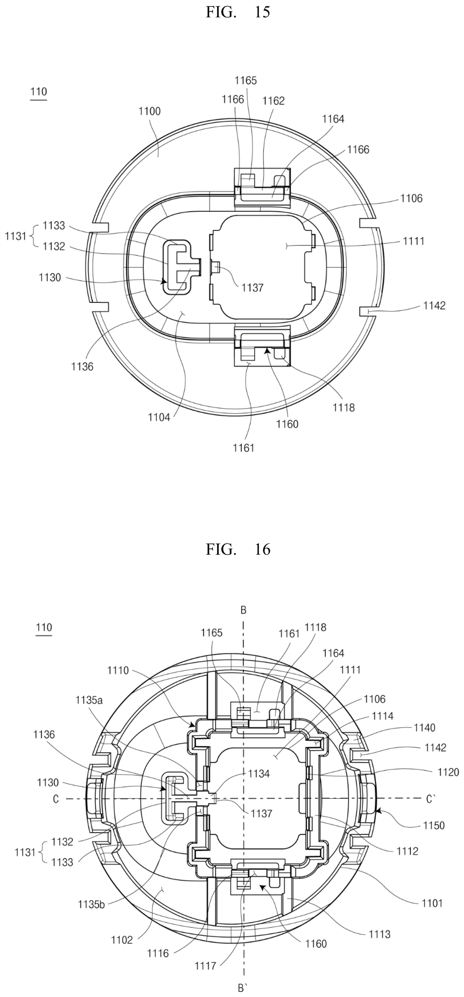

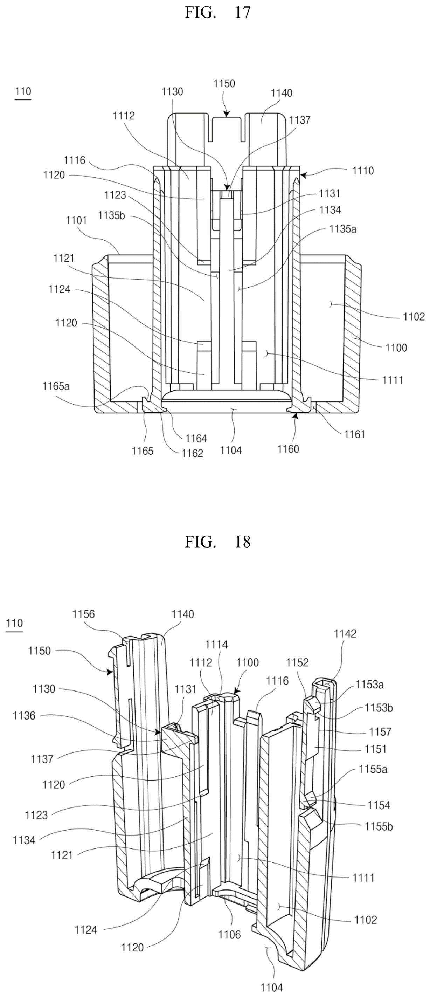

FIG. 14 is a perspective view and a partially enlarged view illustrating an exemplary embodiment of the body housing 110, FIG. 15 is a bottom view illustrating an exemplary embodiment of the body housing 110, and FIG. 16 is a plan view illustrating an exemplary embodiment of the body housing 110. FIG. 17 is a cross-sectional view of the body housing 110 cut along B-B' of FIG. 16, and FIG. 18 is a partial cross-sectional perspective view of the body housing 110 cut along C-C' of FIG. 16.

The body housing 110 may be configured to support a sensor unit carrier 130 disposed in an interior of the applicator 10, guide a movement direction of the sensor unit carrier 130, and restrict a movement range of the sensor unit carrier 130. The transmission unit 40 for attachment to a human body B may be separably and fixedly disposed at one end of the body housing 110.

The body housing 110 may include a body housing body portion 1100 having a circumferential surface of a shape corresponding to the first interior space 1202 of the handle housing 120. A second interior space 1102 may be provided in an interior of the body housing body portion 1100. The second interior space 1102 may be connected to the exterior through a second opening 1101 formed at one end of the body housing 110 adjacent to the handle housing 120.

When the body housing 110 and the handle housing 120 are assembled to be coupled to each other, the second interior space 1102 formed in the body housing 110 and the first interior space 1202 formed in the handle housing 120 may be connected to each other to form an interior space (not illustrated) of the interior of the applicator 10 separated from the exterior. Meanwhile, the other end side of the body housing 110 facing the one end where the second opening 1101 is formed may be provided in a closed form.

In the interior of the body housing 110, a column 1110 in which a first movement space 1111 is formed to penetrate along the first direction may be disposed upright. The column 1110 may be provided at a position corresponding to the carrier fixing fence 1207 provided in the handle housing 120. The column 1110 may include a plurality of partition walls 1112 extending along a direction opposite to the first direction from an inner surface of the closed other end side of the body housing 110 and disposed upright. The plurality of partition walls 1112 may extend from an interior of the second interior space 1102 to the exterior so that end portions of the respective partition walls 1112 protrude outward of the second opening 1101. The plurality of partition walls 1112 may be provided to surround the first movement space 1111 at a side surface side. The second interior space 1102 and the first movement space 1111 may be separated by the plurality of partition walls 1112. A sensor unit carrier body 1310 of the sensor unit carrier 130 to be described later, the needle carrier 140, and the sensor unit 30 may move toward the transmission unit 40 through the first movement space 1111 in an insertion process of the transcutaneous sensor member 330 into subcutaneous tissue.

Meanwhile, although a hexagonal column form of the column 1110 will be described as an example hereinafter, a shape of the column 1110 of the present invention is not limited thereto, and as long as it is a shape capable of providing a movement path of the sensor unit carrier body 1310, the needle carrier 140, and the sensor unit 30 in the insertion process of the transcutaneous sensor member 330 into the subcutaneous tissue, it may be variously modified and applied.

On an outer surface of one or more of the plurality of partition walls 1112, a first acceleration latch 1118 provided in a shape protruding toward the outside from the outer surface of the partition wall 1112 may be provided. The first acceleration latch 1118 may be provided in a shape protruding in a direction away from the first movement space 1111 at an outer region of the first movement space 1111. The first acceleration latch 1118 may be provided at an outer region of the second interior space 1102. The first acceleration latch 1118 may interact with a second acceleration latch 1328 provided in the sensor unit carrier 130 to be described later so as to grant a movement initiation condition of the sensor unit carrier 130 to move in the first direction only when a force of a predetermined amount or more is applied to the sensor unit carrier 130. That is, since insertion of the transcutaneous sensor member 330 into subcutaneous tissue is carried out only when a force sufficient to release a movement constraint of the second acceleration latch 1328 by the first acceleration latch 1118 is applied to the handle housing 120, a situation in which the transcutaneous sensor member 330 is arbitrarily fired in an unintended situation by a user may be effectively avoided.

Meanwhile, for the transcutaneous sensor member 330 to be normally inserted into the subcutaneous tissue, it is required that the needle body 1402 move at a speed equal to or higher than a reference speed so that a leading end of the needle body 1402 penetrates a skin surface. Through interaction between the first acceleration latch 1118 and the second acceleration latch 1328, an acceleration condition may be provided such that the needle body 1402 is triggered at a speed equal to or higher than the reference speed, thereby effectively inducing normal subcutaneous insertion of the transcutaneous sensor member 330.

The first acceleration latch 1118 may be provided as a latch structure having a wedge-shaped cross-section provided with an inclined surface 1119a and a support surface 1119b, but a shape of the first acceleration latch 1118 is not necessarily limited thereto. The support surface 1119b may be provided at a position adjacent to the second opening 1101 relative to the inclined surface 1119a. The first acceleration latch 1118 may be variously modified and applied as long as it is a shape capable of imparting a movement initiation condition or an acceleration condition of the sensor unit carrier 130 through interaction with the second acceleration latch 1328. The first acceleration latch 1118 may be means for granting a movement initiation condition or an acceleration condition of the sensor unit carrier 130 through interaction with the second acceleration latch 1328.

At a leading end portion of the partition wall 1112 provided with the first acceleration latch 1118, a bridge pressing portion 1116 having a shape in which a cross-section decreases toward a leading end may be provided. The bridge pressing portion 1116 may be provided to press a bridge 1330 provided in the sensor unit carrier 130 to be described later so as to deform or cut the bridge 1330. The bridge pressing portion 1116 may be disposed to be spaced apart at a predetermined interval at a position facing the bridge 1330. As the sensor unit carrier 130 moves in the first direction, a distance between the bridge 1330 and the bridge pressing portion 1116 may become closer. That is, in an initial step of movement of the sensor unit carrier 130 in the first direction, the bridge 1330 provided in the sensor unit carrier 130 is moved to a position in close contact with the bridge pressing portion 1116, and only when a force in the first direction sufficient to cut or deform the bridge 1330 pressed by the bridge pressing portion 1116 is applied to the sensor unit carrier 130, movement of the sensor unit carrier 130 in the first direction may be completed.

When the bridge 1330 is not cut or deformed despite the movement of the sensor unit carrier 130 in the first direction, a state in which the bridge 1330 is caught by the bridge pressing portion 1116 is maintained, and the sensor unit carrier 130 may be placed in a situation where the sensor unit carrier 130 can no longer move in the first direction. That is, not only may arbitrary firing of the transcutaneous sensor member 330 in an unintended situation by a user be effectively prevented through interaction between the bridge 1330 and the bridge pressing portion 1116, but also sufficient acceleration conditions for normal insertion of the transcutaneous sensor member 330 into the subcutaneous tissue may be granted. In order to exclude mutual interference between the first acceleration latch 1118 and the second acceleration latch 1328, the bridge 1330 may be disposed at a position not overlapping with the first acceleration latch 1118 and the second acceleration latch 1328 along the first direction. The bridge pressing portion 1116 may be provided in an outer region of the second interior space 1102, and may be disposed at a position protruding further from the second opening 1101 than the first acceleration latch 1118.

During assembly of the applicator assembly 1, as the sensor unit carrier body 1310 is introduced into the first movement space 1111, at least one end portion of the carrier guide protrusion 1310a may be disposed in the interior of the carrier guide groove 1114, and accordingly, the bridge 1330 may be disposed at a position facing the bridge pressing portion 1116.

Between the first acceleration latch 1118 and the bridge pressing portion 1116, a carrier slit 1117 formed to penetrate in a shape incising the partition wall 1112 along a direction parallel to a direction opposite to the first direction from the leading end portion of the partition wall 1112 may be provided. The carrier slit 1117 may more preferably be formed to penetrate between the first acceleration latch 1118 and the bridge pressing portion 1116 in terms of structural simplification. Meanwhile, as illustrated in FIG. 14, a case is shown in which the first acceleration latch 1118, the carrier slit 1117, and the bridge pressing portion 1116 are disposed adjacent to one another, but the structure is not necessarily limited thereto, and the first acceleration latch 1118, the carrier slit 1117, and the bridge pressing portion 1116 may be disposed spaced apart at predetermined intervals.

The carrier slit 1117 and the first movement space 1111 may be in communication, and by the carrier slit 1117, the first movement space 1111 and the second interior space 1102 may be in communication. At an extension arm connection portion 1322 disposed between a sensor unit carrier body 1310 and an extension arm 1320, an extension arm guide groove 1324 recessed in a shape corresponding to the carrier slit 1117 may be formed to extend along the first direction.

During movement of the sensor unit carrier 130 along the first direction, at least one end of the extension arm guide groove 1324 may be introduced into an interior of the carrier slit 1117. As at least one end of the extension arm connection portion 1322 in which the extension arm guide groove 1324 is formed is introduced into the interior of the carrier slit 1117, interference of the extension arm connection portion 1322 in movement along the first direction by the partition wall 1112 may be excluded, and the movement of the sensor unit carrier 130 may be guided by the carrier slit 1117.

As the extension arm connection portion 1322 moves along the carrier slit 1117, the sensor unit 30 separably fixed to an end portion of the sensor unit carrier 130 may be accurately delivered to the seating groove 412 of the transmission unit 40.

In FIG. 14 and FIGS. 17 to 18, a case is exemplarily illustrated in which, after pressing of the bridge 1330 by the bridge pressing portion 1116 is initiated in a process in which the sensor unit carrier 130 moves along the first direction, the bridge 1330 is disposed at a position in which at least one end of the extension arm guide groove 1324 is introduced into the carrier slit 1117. However, it may include a case in which the bridge 1330 is disposed at a position where at least one end of the extension arm guide groove 1324 is introduced into the carrier slit 1117 immediately before pressing of the bridge 1330 by the bridge pressing portion 1116 or simultaneously with pressing of the bridge 1330 by the bridge pressing portion 1116.

The first acceleration latch 1118, the bridge pressing portion 1116, and the carrier slit 1117 may be provided at one side end of the column 1110, and at least one of them may be provided in pairs in a symmetrical shape at both side ends of the column 1110.

On an inner surface of the partition wall 1112 forming the first movement space 1111, a carrier guide groove 1114 may be formed to be recessed in a direction intersecting the first direction, and the carrier guide groove 1114 may be formed to extend along the first direction. For example, the carrier guide groove 1114 may be formed in a shape recessed from the inner surface of the partition wall 1112 so as to protrude toward the second interior space 1102 from the first movement space 1111. The carrier guide groove 1114 may be formed to extend to a height corresponding to an entire height of the partition wall 1112. The carrier guide groove 1114 may be formed in the partition wall 1112 adjacent to the partition wall 1112 in which the first acceleration latch 1118, the bridge pressing portion 1116, and the carrier slit 1117 are formed, and a plurality of the carrier guide grooves 1114 may be formed in one partition wall 1112. The carrier guide groove 1114 may be formed in a region adjacent to the partition wall 1112 in which the first acceleration latch 1118, the bridge pressing portion 1116, and the carrier slit 1117 are formed. The carrier guide groove 1114 may be provided in pairs in a symmetrical shape at both side ends of the column 1110.

The sensor unit carrier 130 may be provided with a carrier guide protrusion 1310a formed in a shape corresponding to the carrier guide groove 1114 at a position corresponding to the carrier guide groove 1114. The carrier guide protrusion 1310a may protrude from one end of the sensor unit carrier body 1310 in a direction intersecting the first direction and may be formed in a shape extending along the first direction. The carrier guide protrusion 1310a may be provided in a corner portion region of the sensor unit carrier body 1310. At least one end of the carrier guide protrusion 1310a may be disposed in the interior of the carrier guide groove 1114. A leading end portion of the carrier guide protrusion 1310a may be disposed in the carrier guide groove 1114 to guide the movement direction of the sensor unit carrier 130 so that the sensor unit carrier 130 moves along the first direction.

In the drawings, four carrier guide protrusions 1310a disposed at respective corner portions of the sensor unit carrier 130 and a corresponding number of carrier guide grooves 1114 formed on the inner surface of the column 1110 at corresponding positions are exemplarily illustrated. However, shapes and numbers of the carrier guide protrusion 1310a and the carrier guide groove 1114 are not necessarily limited to those illustrated in the drawings. The carrier guide protrusion 1310a and the carrier guide groove 1114 may be variously modified and applied as long as they are in shapes and numbers capable of guiding movement of the sensor unit carrier 130 along the first direction.

In the second interior space 1102, a partition wall support portion 1113 provided to support the column 1110 may be disposed upright. The partition wall support portion 1113 may be provided in plurality and may extend in a shape from an interior of the second interior space 1102 to a position adjacent to the second opening 1101. The partition wall support portion 1113 may be disposed so that one end thereof is connected to the partition wall 1112 and the other end thereof is connected to an inner side wall of the body housing body portion 1100, thereby improving structural stability and rigidity of the column 1110 and the body housing body portion 1100.

A body extension portion 1140 may be provided in a shape extending along a direction opposite to the first direction from one end portion of the body housing body portion 1100 in which the second opening 1101 is formed. The body extension portion 1140 may be provided in pairs in a symmetrical shape so as to face each other at the one end portion of the body housing body portion 1100. The body extension portion 1140 may be provided at a position facing the partition wall 1112 in which the carrier guide groove 1114 is formed. The body extension portion 1140 may be provided in a shape extending to a region in which a leading end portion of the body extension portion 1140 protrudes more outward from the second opening 1101 than a leading end portion of the column 1110. The handle housing guide groove 1142 may be formed in a shape extending along the first direction from an outer surface of a leading end portion side of a body extension portion 1140 to an outer surface of the other end portion side of the body housing body portion 1100. That is, the handle housing guide groove 1142 may be formed to be recessed so as to extend from the outer surface of the body extension portion 1140 to the outer surface of the body housing body portion 1100 in a shape terminating the body extension portion 1140 and the body housing body portion 1100. A leading end portion of the guide protrusion 1210 may be disposed in an interior of the handle housing guide groove 1142 so that a movement direction of the handle housing 120 moving along the first direction may be guided. On one body extension portion 1140, a plurality of handle housing guide grooves 1142 may be formed to be recessed so as to extend along the first direction in a state spaced apart from one another.

On a leading end portion side of the body extension portion 1140, the second movement restriction portion 1150 may be provided. On the leading end portion of the body extension portion 1140, a movement restriction latch accommodation portion 1156 having a shape partially incising the body extension portion 1140 along the first direction from the leading end of the body extension portion 1140 may be formed to penetrate. A movement restriction body 1151 may be disposed to be accommodated in the movement restriction latch accommodation portion 1156, and both side end portions on a central portion side of the movement restriction body 1151 may be connected to the body extension portion 1140 by a body connection portion 1157. When two handle housing guide grooves 1142 are recessedly formed in one body extension portion 1140 to extend along the first direction while being spaced apart from each other, the movement restriction latch accommodation portion 1156 may be formed at a position between the adjacent handle housing guide grooves 1142, and the second movement restriction portion 1150 may also be disposed at a position between the adjacent handle housing guide grooves 1142. The movement restriction latch accommodation portion 1156 may be formed to incise the body extension portion 1140 along the first direction up to a region not exceeding the body extension portion 1140, and the handle housing guide groove 1142 may be formed to extend in a shape from an end portion of the body extension portion 1140 to an end portion of the body housing body portion 1100.

Since both side end portions on the central portion side of the movement restriction body 1151 are connected to the body extension portion 1140 through the body connection portion 1157, when an external force is applied to the movement restriction body 1151, the movement restriction body 1151 may be deformed in a state of being accommodated in the movement restriction latch accommodation portion 1156. As the movement restriction body 1151 is deformed, at least one end of the second movement restriction portion 1150 may be displaced and moved toward the first movement space 1111. In the present invention, the term "deformed" is interchangeably used with "bent", "curved", "twisted" (including torsionally deformed) or "distorted". The deformation of the movement restriction body 1151 is temporary and reversible, and when the external force is removed, it returns to its original position and/or shape.

On an outer surface of a one side end portion of the movement restriction body 1151, a first movement restriction latch 1152 may be provided, and on an outer surface of the other side end portion of the movement restriction body 1151, a second movement restriction latch 1154 may be provided. The first movement restriction latch 1152 and the second movement restriction latch 1154 may be provided in shapes protruding toward the second interior space 1102 from an outer region of the first movement space 1111. The first movement restriction latch 1152 and the second movement restriction latch 1154 may preferably be disposed to be spaced apart from each other along the first direction. The body connection portion 1157 may be provided in a region adjacent to the first movement restriction latch 1152 relative to the second movement restriction latch 1154.

The first movement restriction latch 1152 may be provided as a latch structure having a wedge-shaped cross-section provided with a first inclined surface 1153a and a first support surface 1153b. The second movement restriction latch 1154 may also be provided as a latch structure having a wedge-shaped cross-section provided with a second inclined surface 1155a and a second support surface 1155b. Along the first direction, the first inclined surface 1153a, the first support surface 1153b, the second inclined surface 1155a, and the second support surface 1155b may be sequentially disposed. The first movement restriction latch 1152 and the second movement restriction latch 1154 may interact with the first movement restriction portion 1220 of the above-described handle housing 120 to constrain movement of the handle housing 120.

The support surface 1222 of the first movement restriction portion 1220 may be formed along a direction substantially parallel to the second direction, and the inclined surface 1221 of the first movement restriction portion 1220 may be inclinedly disposed so as to become adjacent to an inner side wall of the handle housing 120 along the first direction from an end portion of the protruding support surface 1222. Along the first direction, the support surface 1222 and the inclined surface 1221 may be sequentially disposed. The first support surface 1153b of the first movement restriction latch 1152 may be formed along a direction substantially parallel to the second direction.

The first inclined surface 1153a of the first movement restriction latch 1152 may be disposed inclined from an end portion of the protruding first support surface 1153b along a direction opposite to the first direction so as to become adjacent to one end portion side of the movement restriction body 1151. The second support surface 1155b of the second movement restriction latch 1154 may be formed along a direction substantially parallel to the second direction. The second inclined surface 1155a of the second movement restriction latch 1154 may be disposed inclined from an end portion of the protruding second support surface 1155b along a direction opposite to the first direction so as to become adjacent to the other end portion side of the movement restriction body 1151.

When the handle housing 120 and the body housing 110 are assembled, in a process in which the handle housing 120 relatively moves with respect to the body housing 110 in the first direction, the inclined surface 1221 of the first movement restriction portion 1220 may reach a state of coming into contact with the first inclined surface 1153a of the first movement restriction latch 1152. Thereafter, as the handle housing 120 further moves along the first direction, one end of a movement restriction body 1151 may be deformed toward the second interior space 1102, and accordingly, the first movement restriction portion 1220 may pass through the first movement restriction latch 1152.

After the first movement restriction portion 1220 passes through the first movement restriction latch 1152, the movement restriction body 1151 may be restored to a state before deformation. After the first movement restriction portion 1220 passes through the first movement restriction latch 1152 and coupling of the handle housing 120 and the body housing 110 is completed, since the support surface 1222 of the first movement restriction portion 1220 and the first support surface 1153b of the first movement restriction latch 1152 are placed in a state of facing each other, even if a force in a direction opposite to the first direction is applied to the handle housing 120, the support surface 1222 of the first movement restriction portion 1220 and the first support surface 1153b of the first movement restriction latch 1152 may support each other, thereby preventing the handle housing 120 from being arbitrarily deviated from the body housing 110.

In an insertion process of the transcutaneous sensor member 330 into the subcutaneous tissue, in a state in which the inclined surface 1221 of the first movement restriction portion 1220 comes into contact with the second inclined surface 1155a of the second movement restriction latch 1154, the handle housing 120 may move in the first direction, and the other end of the movement restriction body 1151 may be deformed toward the second interior space 1102 so that the first movement restriction portion 1220 passes through the second movement restriction latch 1154. After the first movement restriction portion 1220 passes through the second movement restriction latch 1154, the movement restriction body 1151 may be restored to a state before deformation.

After the first movement restriction portion 1220 passes the second movement restriction latch 1154 and insertion of the transcutaneous sensor member 330 into the subcutaneous tissue is completed, the support surface 1222 of the first movement restriction portion 1220 and the second support surface 1155b of the second movement restriction latch 1154 may be disposed to face each other. By interaction between the support surface 1222 of the first movement restriction portion 1220 and the second support surface 1155b of the second movement restriction latch 1154, movement of the handle housing 120 in a direction opposite to the first direction may be restricted. That is, after the insertion of the transcutaneous sensor member 330 into the subcutaneous tissue, through interaction between the first movement restriction portion 1220 and the second movement restriction portion 1150, restoration of the handle housing 120 to a position before insertion of the transcutaneous sensor member 330 may be restricted, and accordingly, reuse of the applicator 10 after firing may be effectively prevented.

In one of the plurality of partition walls 1112, the fixing portion 1130 for restricting arbitrary movement of the sensor unit carrier 130 during assembly of the applicator 10 may be provided. The fixing portion 1130 may preferably be provided in one of the partition walls 1112 in which the carrier guide groove 1114 is formed. In a leading end portion of the partition wall 1112 provided with the fixing portion 1130, a first incision groove 1135a and a second incision groove 1135b formed to incise the partition wall 1112 along the first direction from a leading end of the partition wall 1112 may be formed to penetrate in parallel while being spaced apart from each other.

Between the first incision groove 1135a and the second incision groove 1135b, the support 1134 may be disposed upright along a direction parallel to the first direction. A fixing protrusion portion 1137 having a shape protruding toward the first movement space 1111 may be provided on one surface of the support 1134 facing the first movement space 1111. On the other surface of the support 1134, opposite to the one surface on which the fixing protrusion portion 1137 is formed, a pressing movement extension portion 1136 extending from the other surface of the support 1134 may be provided. At a protruding leading end portion of the pressing movement extension portion 1136, a pressing movement portion 1131 may be provided. The fixing protrusion portion 1137 and the pressing movement extension portion 1136 may be disposed at symmetrical positions at a leading end portion of the support 1134. The fixing protrusion portion 1137 may be positioned in a region closer to the second opening 1101 compared to the bridge pressing portion 1116.

The pressing movement portion 1131 may include a pressing movement body portion 1132 connected to the pressing movement extension portion 1136, and pressing movement wing portions 1133 provided at both side ends of the pressing movement body portion 1132. The pressing movement wing portion 1133 may be provided with a pressing movement inclined surface 1133a having a shape coming close to the partition wall 1112 along the first direction. By the pressing movement body portion 1132 disposed to connect the pressing movement extension portion 1136 and the pressing movement wing portion 1133, the pressing movement wing portion 1133 may be disposed at a position spaced apart from the pressing movement extension portion 1136. The pressing movement extension portion 1136, the pressing movement body portion 1132, and the pressing movement wing portion 1133 may be formed to have substantially corresponding heights. As described below, on one surface of the sensor unit carrier body 1310, a fixing groove 1340 having a shape corresponding to the fixing protrusion portion 1137 may be formed to be recessed.

When defining a position of the sensor unit carrier 130 in a state where the handle housing 120 is not pressed by a user (that is, a state where firing of the transcutaneous sensor member 330 and insertion into subcutaneous tissue are not performed) as an initial position, and defining a position of the sensor unit carrier 130 in a state after the handle housing 120 is pressed by the user (that is, a state after firing of the transcutaneous sensor member 330 and insertion into subcutaneous tissue are performed) as an insertion position, it may be preferable that the fixing groove 1340 is formed to be recessed on one surface of the sensor unit carrier body 1310 so that the fixing groove 1340 of the sensor unit carrier 130 positioned at the initial position and the fixing protrusion portion 1137 are positioned at a position corresponding to each other.

In a process of fitting the sensor unit carrier body 1310 into the first movement space 1111 in order to assemble the applicator 10, the fixing protrusion portion 1137 comes into contact with one surface of the sensor unit carrier body 1310, whereby the support 1134 is deformed outward, and the fixing portion 1130 is maintained in a state of being moved in a direction away from the first movement space 1111. Subsequently, when the sensor unit carrier body 1310 is pushed into the first movement space 1111 along the first direction until the fixing protrusion portion 1137 and the fixing groove 1340 correspond to each other in position, a leading end portion of the fixing protrusion portion 1137 is introduced into and disposed in an interior of the fixing groove 1340, and the support 1134 is restored to a state before deformation.

As the leading end portion of the fixing protrusion portion 1137 is introduced into the interior of the fixing groove 1340, arbitrary movement of the sensor unit carrier 130 in the first direction or in a direction opposite to the first direction is restricted, and subsequent coupling work of the handle housing 120 and the body housing 110 may be performed.

In a process of coupling the handle housing 120 to the body housing 110, a leading end portion of the push arm 1230 provided on the handle housing 120 may come into contact with the pressing movement inclined surface 1133a of the pressing movement wing portion 1133. As a leading end portion of the push arm 1230 comes into contact with the pressing movement inclined surface 1133a and moves in the first direction to press the pressing movement wing portion 1133, the support 1134 may be deformed outward, and the pressing movement portion 1131 may be displaced in a direction away from the sensor unit carrier body 1310. As the pressing movement portion 1131 is pushed in a direction away from the sensor unit carrier body 1310, the leading end portion of the fixing protrusion portion 1137 moves to a position deviated from the fixing groove 1340, and accordingly, the arbitrary movement restriction of the sensor unit carrier 130 by the fixing portion 1130 may be released. Since assembly of the applicator 10 may be performed in a state where the sensor unit carrier 130 maintains the initial position by the fixing portion 1130, work efficiency during the assembly work of the applicator 10 may be more effectively improved.

Meanwhile, although in the above, a case where the leading end portion of the push arm 1230 presses one end of the fixing portion 1130 in an assembly process of the applicator 10 so that the fixing protrusion portion 1137 is discharged from the fixing groove 1340 has been described as an example, it may include a case where in a firing process of the transcutaneous sensor member 330, the leading end portion of the push arm 1230 presses one end of the fixing portion 1130 so that the fixing protrusion portion 1137 is discharged from the fixing groove 1340. That is, in the assembly process of the applicator 10, the leading end portion of the push arm 1230 may be positioned at a position adjacent to one end of the fixing portion 1130 or at a position in contact with one end of the fixing portion 1130, and in a process in which a user presses the handle housing 120 so that the handle housing 120 moves from the first position to the second position, the fixing protrusion portion 1137 may be discharged from the fixing groove 1340 by the leading end portion of the push arm 1230 pressing one end of the fixing portion 1130.

On a closed one end side of the body housing 110 facing the second opening 1101, a transmission unit accommodation portion 1104 capable of accommodating the transmission unit 40 may be provided. The transmission unit accommodation portion 1104 may be formed in a shape recessed from an exterior of the body housing 110 toward the second interior space 1102, and the transmission unit accommodation portion 1104 may be physically separated from the second interior space 1102. The transmission unit accommodation portion 1104 may be provided in a region including a central portion of a closed one end side of the body housing 110. The transmission unit accommodation portion 1104 may be formed to be recessed in a shape corresponding to the transmission unit housing 410 so that the transmission unit housing 410 can be accommodated therein. In a state where the transmission unit 40 is accommodated in the transmission unit accommodation portion 1104, when one end of the applicator 10 is brought into close contact with skin, the transmission unit accommodation portion 1104 may be preferably formed to be recessed to a recessed depth such that the adhesive member 430 provided in the transmission unit 40 may be attached to the skin.

At one end of the body housing 110 where the transmission unit accommodation portion 1104 is formed, a third opening 1106 connecting the transmission unit accommodation portion 1104 and the first movement space 1111 may be formed to penetrate. Through the third opening 1106, the transmission unit accommodation portion 1104 and the first movement space 1111 may be connected. The third opening 1106 may be formed to have an area smaller than that of a region in which the transmission unit accommodation portion 1104 is formed. The transmission unit 40 may be disposed in the transmission unit accommodation portion 1104 such that the seating groove 412 formed on one surface of the transmission unit 40 is positioned at a position corresponding to the third opening 1106. In an insertion process of the transcutaneous sensor member 330 into the subcutaneous tissue, the sensor unit 30 that has moved along the first direction from the first movement space 1111 may pass through the third opening 1106 and be delivered to the seating groove 412 of the transmission unit 40 accommodated in the transmission unit accommodation portion 1104.

At both side ends of the transmission unit accommodation portion 1104, transmission unit support portions 1160 for preventing arbitrary deviation of the transmission unit 40 from the transmission unit accommodation portion 1104 may be provided. The transmission unit support portion 1160 may be disposed at a position adjacent to the third opening 1106. A pair of transmission unit support portions 1160 may be disposed respectively at one side of the first movement space 1111 and at a radially outer side opposite thereto. The fixing portion 1130 may be disposed at an outer side of the first movement space 1111 opposite to the pair of transmission unit support portions 1160, without overlapping the pair of transmission unit support portions 1160. Meanwhile, the number of the transmission unit support portions 1160 is not particularly limited, but in a case where the transmission unit support portions 1160 are provided in pairs, it may be more effective in preventing deviation of the transmission unit 40 and ensuring stable carrying of the applicator assembly 1. The transmission unit support portion 1160 may be disposed in a region adjacent to the partition wall 1112 in which the first acceleration latch 1118, the bridge pressing portion 1116, and the carrier slit 1117 are provided. The transmission unit support portion 1160 may be formed integrally with the body housing body portion 1100 by injection molding.

At both side ends of the transmission unit accommodation portion 1104, support hook accommodation spaces 1161 formed to penetrate a closed one end of the body housing 110 may be provided. A transmission unit support hook 1162 configured to support the transmission unit 40 may be positioned in the support hook accommodation space 1161. The transmission unit support hook 1162 may be supported by a pair of support hook connection portions 1166, which extend respectively from an end portion of the body housing 110 forming the support hook accommodation space 1161 and are connected to both side ends of the transmission unit support hook 1162. The pair of support hook connection portions 1166 may be disposed in a region partially overlapping with the partition wall 1112 in which the first acceleration latch 1118, the bridge pressing portion 1116, and the carrier slit 1117 are provided. Since the transmission unit support hook 1162 is connected to the support hook connection portion 1166 extending from the end portion of the body housing 110 and disposed in the support hook accommodation space 1161, when an external force is applied to the transmission unit support hook 1162, the support hook connection portion 1166 may be deformed so that the position of the transmission unit support hook 1162 may be changed.

In FIGS. 15 and 16, the support hook accommodation space 1161 is illustrated in a form having a substantially rectangular cross-section. However, the shape of the support hook accommodation space 1161 is not necessarily limited thereto. The support hook accommodation space 1161 may be formed in a cross-sectional shape corresponding to that of the transmission unit support hook 1162. As a non-limiting example, the support hook accommodation space 1161 may have a cross-sectional shape similar to a "Γ"-shape or a "Π"-shape, and a pair of support hook accommodation spaces 1161 may be provided symmetrically with respect to the first movement space 1111.

The transmission unit support hook 1162 may include a catching portion 1164 formed to protrude in a direction toward the transmission unit accommodation portion 1104 and a pushed portion 1165 formed to protrude in a direction opposite to the transmission unit accommodation portion 1104. The catching portion 1164 may be formed in a shape protruding toward the first movement space 1111, and the pushed portion 1165 may be formed in a shape protruding toward the second interior space 1102. The catching portion 1164 and the pushed portion 1165 may be provided in shapes extending in opposite directions to each other. The pushed portion 1165 may be provided to have a width equal to or smaller than that of the catching portion 1164 (wherein the width of the pushed portion 1165 and the catching portion 1164 may mean the width in a lateral direction based on what is illustrated in FIGS. 15 and 16). The pushed portion 1165 may be disposed at a position eccentrically shifted to one side relative to the overall width of the catching portion 1164. The catching portion 1164 may be provided in a region overlapping with a region where the first acceleration latch 1118, the bridge pressing portion 1116, and the carrier slit 1117 are disposed, and the pushed portion 1165 may be provided in a region overlapping with a region where the bridge pressing portion 1116 is disposed.

A leading end portion of the catching portion 1164 may be inserted into the transmission unit housing groove 417 of the transmission unit 40 accommodated in the transmission unit accommodation portion 1104. The transmission unit 40 accommodated in the transmission unit accommodation portion 1104 may be supported by the catching portion 1164, with the leading end portion inserted into the transmission unit housing groove 417, and arbitrary deviation from the transmission unit accommodation portion 1104 may thereby be prevented. The catching portion 1164 may have a shape in which the cross-section decreases toward the leading end portion.

The pushed portion 1165 may be provided to have a height greater than that of the catching portion 1164. At one end of the pushed portion 1165 facing the extension arm pushing portion 1326, a pushed portion inclined surface 1165a inclined in a shape approaching the transmission unit accommodation portion 1104 along the first direction may be formed. That is, since the pushed portion inclined surface 1165a is provided at one end of the pushed portion 1165 facing the extension arm pushing portion 1326, the one end portion of the pushed portion 1165 facing the extension arm pushing portion 1326 may have a shape in which the cross-section decreases in a direction opposite to the first direction. By pressing the pushed portion inclined surface 1165a with an extension arm pushing portion 1326 of the sensor unit carrier 130 to be described later, the transmission unit support hook 1162 may be moved in a direction away from the transmission unit accommodation portion 1104. That is, in the insertion process of the transcutaneous sensor member 330 into the subcutaneous tissue, the sensor unit carrier 130 moves together with the sensor unit 30 along the first direction, and at the same time when the sensor unit 30 is delivered from the sensor unit carrier 130 to the transmission unit 40, or at a point in time somewhat earlier than a point in time when the sensor unit 30 is delivered from the sensor unit carrier 130 to the transmission unit 40, the extension arm pushing portion 1326 may press the pushed portion inclined surface 1165a so that movement restriction of the transmission unit 40 by the catching portion 1164 may be released. In a case after delivery of the sensor unit 30 to the transmission unit 40, the spacing width between opposing catching portions 1164 may be wider than in a case before the sensor unit 30 is delivered to the transmission unit 40.

During manufacturing of the applicator assembly 1, in a process of disposing the transmission unit 40 in the transmission unit accommodation portion 1104, the transmission unit support hook 1162 may be pressed by the transmission unit housing 410 and may be moved in a direction away from the transmission unit accommodation portion 1104. When the transmission unit 40 is completely seated in the transmission unit accommodation portion 1104, the moved transmission unit support hook 1162 may be restored, and accordingly, a leading end portion of the catching portion 1164 may be introduced into the transmission unit housing groove 417 and be disposed therein.

The transmission unit support portion 1160 is provided so as to release movement constraint of the transmission unit 40 only when intended by a worker or a user in an assembly process or a using process of the applicator assembly 1, and thus, assembling work convenience and convenience of use of the applicator assembly 1 may be more effectively improved.

On an inner surface of at least one of the partition walls 1112 forming the first movement space 1111, a grip arm guide groove 1120 extending along the first direction may be formed in a shape recessed outward. The grip arm guide groove 1120 may be formed in a partition wall 1112 neighboring a partition wall 1112 in which the first acceleration latch 1118, the bridge pressing portion 1116, and the carrier slit 1117 are provided. A grip arm 1422 of the needle carrier 140 to be described later may be disposed in the grip arm guide groove 1120 so that a movement direction of the needle carrier 140 may be guided.

At a central portion of the grip arm guide groove 1120, a step portion 1121 protruding to a height substantially corresponding to one surface of the partition wall 1112 in which the grip arm guide groove 1120 is formed may be formed, and at one end and the other end of the step portion 1121, a first step inclined surface 1123 and a second step inclined surface 1124 may respectively be formed. That is, on the inner surface of the partition wall 1112, the grip arm guide groove 1120, the first step inclined surface 1123, the step portion 1121, the second step inclined surface 1124, and the grip arm guide groove 1120 may be continuously formed along the first direction. The first step inclined surface 1123 may be inclined in a shape approaching the first movement space 1111 along the first direction. The second step inclined surface 1124 may be inclined in a shape moving away from the first movement space 1111 along the first direction. In a case of the partition wall 1112 in which the fixing portion 1130 is provided, in a region adjacent to the first incision groove 1135a and the second incision groove 1135b, the grip arm guide groove 1120, the first step inclined surface 1123, the step portion 1121, the second step inclined surface 1124, and the grip arm guide groove 1120 may be continuously formed along the first direction, respectively, and the grip arm guide groove 1120, the first step inclined surface 1123, the step portion 1121, the second step inclined surface 1124, and the grip arm guide groove 1120 may be formed in a shape symmetrical with respect to the support 1134.

During assembly of the applicator assembly 1, the needle carrier 140 may be introduced into an interior of the first movement space 1111 along the first direction, and in a state where the grip arm 1422 is disposed in the grip arm guide groove 1120, the needle carrier 140 may move to an initial position. When the needle carrier 140 is disposed in the initial position, a grip arm protrusion portion 1424 formed on the grip arm 1422 may pass through the first step inclined surface 1123 or be positioned at a position immediately before passing through the first step inclined surface 1123. Thereafter, as the needle carrier 140 further moves along the first direction, the grip arm protrusion portion 1424 may come into close contact with the step portion 1121 and may be pressed inward. The sensor unit 30 may be maintained in a more firmly fixed state to the inwardly pressed grip arm 1422 during movement of the needle carrier 140.

In a process in which the needle carrier 140 further moves along the first direction to move to an insertion position, the grip arm protrusion portion 1424 may pass through the second step inclined surface 1124, and as the grip arm protrusion portion 1424 passes through the second step inclined surface 1124, pressing of the grip arm protrusion portion 1424 by the step portion 1121 may be released. That is, in the insertion process of the transcutaneous sensor member 330 into the subcutaneous tissue, the pressing of the grip arm protrusion portion 1424 by the step portion 1121 may be released simultaneously with delivering the sensor unit 30 to the seating groove 412 of the transmission unit 40 or before delivering the sensor unit 30 to the seating groove 412 of the transmission unit 40.

It is possible not only to effectively prevent the sensor unit 30 from being deviated from a designated position in an interior of the applicator 10 during the insertion process of the transcutaneous sensor member 330 into the subcutaneous tissue, but also to effectively prevent a phenomenon in which the sensor unit 30 is brought along in a direction opposite to the first direction by the grip arm 1422 immediately after the insertion of the transcutaneous sensor member 330 into the subcutaneous tissue.

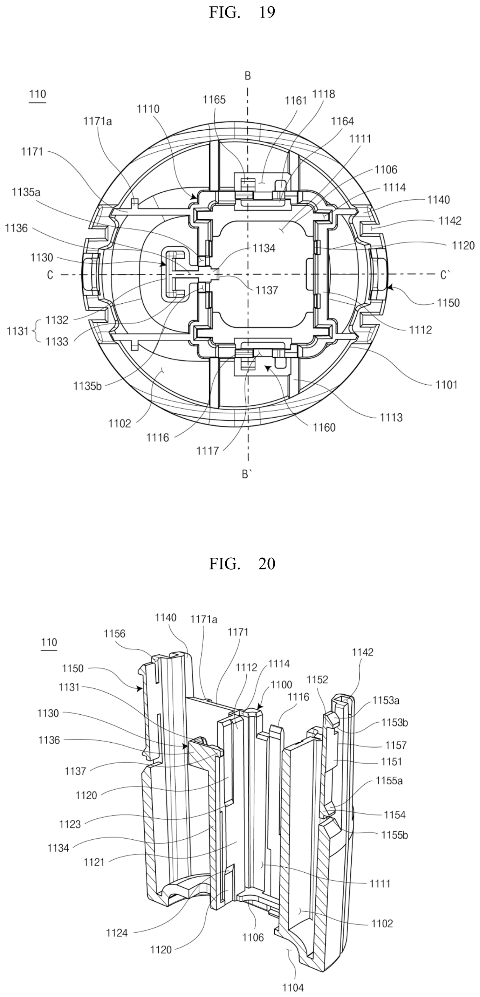

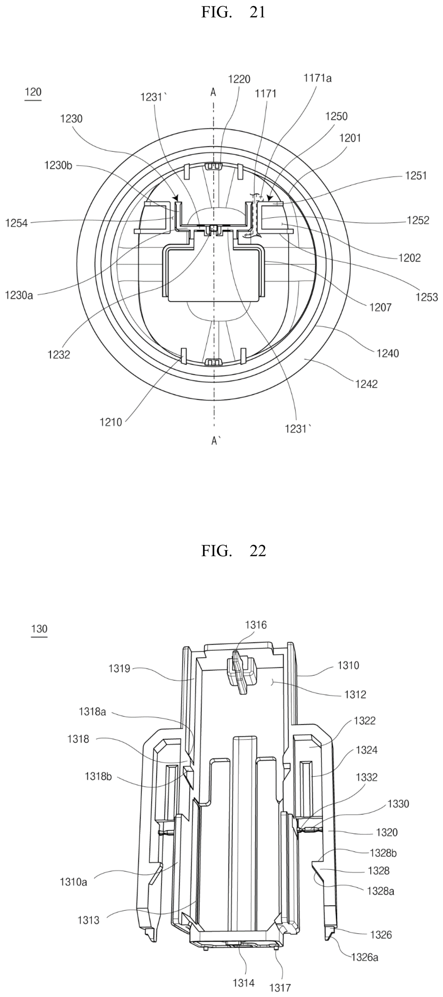

FIG. 19 is a plan view illustrating an exemplary embodiment of the body housing 110 provided with an auxiliary plate 1171, FIG. 20 is a partial sectional perspective view of the body housing 110 cut along C-C' of FIG. 19, and FIG. 21 is a bottom view additionally illustrating a position of the auxiliary plate 1171 in FIG. 12 to explain a positional relationship between the guide frame 1250 and the auxiliary plate 1171.

The body housing 110 may further include a first auxiliary plate 1171 and a second auxiliary plate 1172 provided to connect one end and the other end of the column 1110 and an inner wall of the body housing body portion 1100 and the body extension portion 1140 facing the one end and the other end of the column 1110. The first auxiliary plate 1171 may be disposed to connect one side end of the column 1110 where the fixing portion 1130 is formed and an inner wall of the body housing body portion 1100 and the body extension portion 1140 facing the one side end. The second auxiliary plate 1172 may be disposed at a position opposite to the first auxiliary plate 1171 with respect to the column 1110 so as to connect the other side end of the column 1110 and an inner wall of the body housing body portion 1100 and the body extension portion 1140 facing the other side end. The first auxiliary plate 1171 and the second auxiliary plate 1172 may be disposed on the same line, and the first auxiliary plate 1171 may be provided to have a larger width than the second auxiliary plate 1172. The first auxiliary plate 1171 and the second auxiliary plate 1172 may each extend and be erected in a direction opposite to the first direction from an inner surface of the other closed end side of the body housing 110. The first auxiliary plate 1171 and the second auxiliary plate 1172 may each extend and be erected in a direction opposite to the first direction from an inner surface of the other closed end side of the body housing 110, so as to have a height corresponding to the column 1110.

The first auxiliary plate 1171 may be disposed to connect one side end of the partition wall 1112 where the fixing portion 1130 is formed and an inner wall of the body housing body portion 1100 and the body extension portion 1140 facing the one side end. The second auxiliary plate 1172 may be disposed to connect one side end of the partition wall 1112 opposite to the partition wall 1112 where the fixing portion 1130 is formed and an inner wall of the body housing body portion 1100 and the body extension portion 1140 facing the one side end. The first auxiliary plate 1171 and the second auxiliary plate 1172 may each be provided in pairs. The first auxiliary plates 1171 may each be disposed to connect an outer side end of the partition wall 1112 corresponding to the carrier guide groove 1114 formed adjacent to the fixing portion 1130, and an inner wall of the body housing body portion 1100 and the body extension portion 1140 facing the outer side end. The second auxiliary plates 1172 may each be disposed to connect an outer side end of the partition wall 1112 corresponding to the carrier guide groove 1114 formed in the partition wall 1112 opposite to the partition wall 1112 where the fixing portion 1130 is formed, and an inner wall of the body housing body portion 1100 and the body extension portion 1140 facing the outer side end. A pair of the first auxiliary plates 1171 and a pair of the second auxiliary plates 1172 may be disposed in parallel with each other at positions spaced apart by a predetermined interval. The first auxiliary plate 1171 and the second auxiliary plate 1172, which are symmetrically disposed with respect to the column 1110, may be disposed on the same line.

Since the column 1110 may be supported by the first auxiliary plate 1171 and the second auxiliary plate 1172, which are disposed to connect one end and the other end of the column 1110 and the body housing body portion 1100 and the body extension portion 1140 facing the one end and the other end, the column 1110 may have a more rigid support structure.

On side ends of the first auxiliary plates 1171, protrusion portions 1171a may each be provided to protrude in a direction parallel to the partition wall 1112 where the fixing portion 1130 is formed. The protrusion portions 1171a may each be formed to protrude in a direction away from each other from a side end surface of the first auxiliary plate 1171 outward of a region where the fixing portion 1130 is positioned. The protrusion portion 1171a may be formed to extend along the first direction from an end portion region of the first auxiliary plate 1171 protruding outward of the second interior space 1102. The protrusion portion 1171a may be formed to extend from an end portion region of the first auxiliary plate 1171 to an inner surface of the other closed end side of the body housing 110. In a state where the body housing 110 and the handle housing 120 are coupled, one side surface of the first support plate 1251 facing the protrusion portion 1171a may be disposed in close contact with or in a closely adjacent position to a side end surface of the protrusion portion 1171a. Meanwhile, in a state where the body housing 110 and the handle housing 120 are coupled, one side surface and the other surface of the first support plate 1251 may be disposed in close contact with or in a closely adjacent position to one side surface of the guide plate 1252 and one side surface of the extension push arm plate 1230b.

Through interaction between the guide frame 1250 and the first auxiliary plate 1171, when the body housing 110 and the handle housing 120 are coupled, an end portion of the first auxiliary plate 1171 may be assisted to be introduced into an interior of the guide space 1254, so that the body housing 110 and the handle housing 120 may be coupled at a predetermined coupling position. In a firing process of the transcutaneous sensor member 330, since at least one end of the first auxiliary plate 1171 is maintained in a state disposed in the interior of the guide space 1254, the handle housing 120 may be effectively prevented from moving in a direction deviating from the first direction.

### Sensor unit carrier

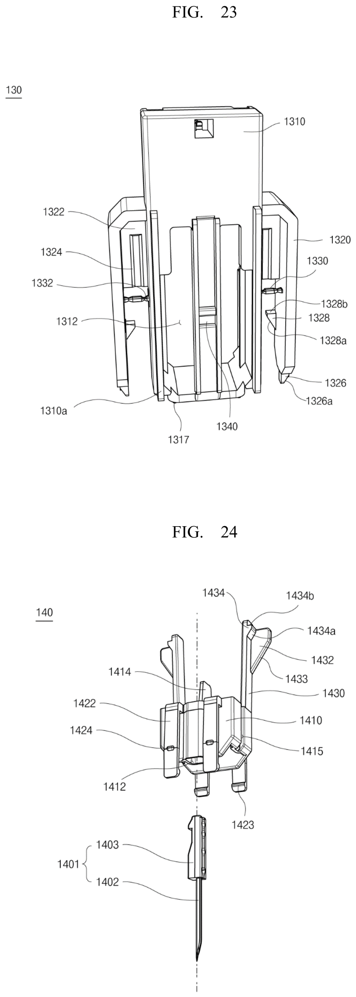

FIG. 22 and FIG. 23 are perspective views illustrating an exemplary embodiment of the sensor unit carrier 130.

The sensor unit carrier 130 may be disposed in an interior of the applicator assembly 1 and is configured to move together with the needle carrier 140 and the sensor unit 30 in the first direction in the insertion process of the transcutaneous sensor member 330 into the subcutaneous tissue. After the insertion of the transcutaneous sensor member 330 into the subcutaneous tissue, the needle carrier 140 may move to a retracted position such that the needle body 1402 is discharged from the skin, whereas the sensor unit carrier 130 may be maintained at an insertion position.

The sensor unit carrier 130 includes the sensor unit carrier body 1310 constituting a skeleton of the sensor unit carrier 130, and the sensor unit carrier body 1310 may be provided with a second movement space 1312 having a front side opened. In a state where the sensor unit carrier 130 is disposed in the column 1110, an open front of the second movement space 1312 may be disposed to face the partition wall 1112 opposite to the fixing portion 1130. The needle carrier 140 may move to the retracted position through the second movement space 1312 after the insertion of the transcutaneous sensor member 330 into the subcutaneous tissue.

At one end portion of the sensor unit carrier body 1310 adjacent to the third opening 1106 formed in the body housing 110, a fourth opening 1314 connecting the exterior and the second movement space 1312 may be formed to penetrate. The needle body 1402 may protrude to the outside of the sensor unit carrier body 1310 through the fourth opening 1314.

On one end surface of the sensor unit carrier body 1310 facing the third opening 1106, a fixing protrusion 1317 having a shape protruding toward the third opening 1106 may be provided, and the sensor unit 30 may be more firmly fixed to the sensor unit carrier 130 by a leading end portion of the fixing protrusion 1317 inserted into the interior of the fixing groove 317 formed in the sensor unit 30. The fixing protrusion 1317 may be disposed at a corner portion of one end surface of the sensor unit carrier body 1310, and may be provided in plurality and disposed at symmetrical positions.

At a corner portion on a side end side of the sensor unit carrier body 1310, the carrier guide protrusion 1310a having a shape protruding toward the outside may be formed to extend along the first direction. With respect to a state where the sensor unit carrier 130 is disposed in the column 1110, the carrier guide protrusion 1310a may be formed to protrude toward the partition wall 1112 in which the carrier guide groove 1114 is formed and extend along the first direction. The carrier guide protrusion 1310a disposed at an open front side of the second movement space 1312 may be provided to have a shorter length than the carrier guide protrusion 1310a disposed opposite thereto. The carrier guide protrusion 1310a disposed opposite to the open front side of the second movement space 1312 may be provided to extend further in a direction opposite to the first direction compared to the carrier guide protrusion 1310a disposed at the open front side of the second movement space 1312. A leading end portion of the carrier guide protrusion 1310a may be disposed in the interior of the carrier guide groove 1114 formed in the column 1110. Through the carrier guide protrusion 1310a and the carrier guide groove 1114, a movement direction of the sensor unit carrier body 1310 moving along the first direction may be guided.

On an inner side surface of the sensor unit carrier body 1310 forming the second movement space 1312, a needle carrier guide protrusion 1313 formed to protrude from the inner side surface of the sensor unit carrier body 1310 toward a central portion of the second movement space 1312 and to extend along the first direction may be provided. The needle carrier guide protrusion 1313 may be provided to have a longer length than the carrier guide protrusion 1310a disposed at the open front side of the second movement space 1312, but may be provided to have a shorter length than the carrier guide protrusion 1310a disposed at a position opposite to the open front side of the second movement space 1312. A leading end portion of the needle carrier guide protrusion 1313 may be disposed in the interior of a needle guide groove 1415 formed in a needle carrier body 1410 to be described later. Through the needle carrier guide protrusion 1313 and the needle guide groove 1415, a movement direction of the needle carrier 140 moving in a direction opposite to the first direction may be guided.

On an inner side surface of the sensor unit carrier body 1310 adjacent to the handle housing 120, a sliding groove 1319 formed to be recessed from an inner surface on a leading end side of the sensor unit carrier body 1310 and to extend along the first direction may be provided. It may be preferable that the sliding groove 1319 is formed at a position adjacent to an opened front surface side of the second movement space 1312. The sliding groove 1319 and the needle carrier guide protrusion 1313 may be disposed at positions spaced apart from each other along the first direction and may be disposed at positions not overlapping with each other.

At an end portion region of the sliding groove 1319 in the first direction, a sensor unit carrier detent 1318 provided with a detent inclined surface 1318a and a detent constraint surface 1318b may be disposed. Since a needle carrier latch 1434 of the needle carrier 140 to be described later maintains a state of being constrained to the sensor unit carrier detent 1318, the needle carrier 140 may move together with the sensor unit carrier 130 in the first direction in the insertion process of a transcutaneous sensor member 330 into the subcutaneous tissue. The detent constraint surface 1318b may be formed along a direction substantially parallel to the second direction, and the detent inclined surface 1318a may be inclinedly disposed so as to be adjacent to the sliding groove 1319 along a direction opposite to the first direction from a protruding one end of the detent constraint surface 1318b.

An extension arm 1320 may be provided in a form of protruding toward the exterior from both outer surfaces of the sensor unit carrier body 1310 and extending along the first direction. The extension arm 1320 may extend outward from a side end of the sensor unit carrier body 1310 in a region overlapping the sliding groove 1319, and may be provided in a pair to form a symmetrical shape relative to the sensor unit carrier body 1310. Between the sensor unit carrier body 1310 and the extension arm 1320, an extension arm connection portion 1322 may be provided to form a closed region. The extension arm connection portion 1322 may extend from a region overlapping the sliding groove 1319 with respect to the second direction toward a region overlapping the needle carrier guide protrusion 1313. An extension arm guide groove 1324 may be recessed in the extension arm connection portion 1322 to extend along the first direction. The extension arm guide groove 1324 may extend to an end portion of the extension arm connection portion 1322 disposed in a region overlapping the needle carrier guide protrusion 1313 with respect to the second direction. A pair of extension arm guide grooves 1324 may be formed in a symmetrical shape on opposite surfaces of the extension arm connection portion 1322. It may be preferable that the extension arm guide groove 1324 is formed in a position and shape corresponding to the carrier slit 1117 of the column 1110.

In order to insert a transcutaneous sensor member 330 into the subcutaneous tissue, in the process in which a sensor unit carrier body 1310 moves along the first direction, an extension arm connection portion 1322 in which an extension arm guide groove 1324 is formed may be introduced into a carrier slit 1117 of a column 1110. By the carrier slit 1117 and the extension arm guide groove 1324, not only the movement direction of the sensor unit carrier 130 moving in the first direction may be guided, but also interference of the first direction movement of the sensor unit carrier 130 by the column 1110 may be excluded.

On one surface of the extension arm 1320 facing the sensor unit carrier body 1310, the second acceleration latch 1328 formed to protrude toward the sensor unit carrier body 1310 may be provided. The second acceleration latch 1328 may be disposed in a region overlapping with the needle carrier guide protrusion 1313 with respect to the second direction. The second acceleration latch 1328 may be formed in a symmetrical shape so as to protrude toward the sensor unit carrier body 1310 from each of the extension arms 1320. The second acceleration latch 1328 may include a support surface 1328b formed along a direction substantially parallel to the second direction, and an inclined surface 1328a inclinedly disposed so as to be gradually adjacent toward the one surface of the extension arm 1320 along the first direction from a protruding end portion of the support surface 1328b. The second acceleration latch 1328 is formed to protrude from one surface of the extension arm 1320 facing the sensor unit carrier body 1310, but the second acceleration latch 1328 may be formed to protrude at a position and size such that a leading end portion of the second acceleration latch 1328 facing the sensor unit carrier body 1310 does not contact the sensor unit carrier body 1310 even by deformation of the shape of the extension arm 1320. The second acceleration latch 1328 may prevent arbitrary firing of the applicator 10 in a situation unintended by a user through interaction with a first acceleration latch 1118 provided in a body housing 110.

The second acceleration latch 1328 may provide sufficient acceleration conditions to a needle body 1402 during subcutaneous tissue insertion of a transcutaneous sensor member 330 through interaction with the first acceleration latch 1118 provided in the body housing 110, and may prevent the sensor unit carrier 130 from moving in a direction opposite to the first direction after the subcutaneous tissue insertion of the transcutaneous sensor member 330.

When the sensor unit carrier 130 is positioned at an initial position, the inclined surface 1119a of the first acceleration latch 1118 and the inclined surface 1328a of the second acceleration latch 1328 may be maintained in a state spaced apart from each other while facing each other, or in a state in contact with each other. When a user presses the handle housing 120 in the first direction so that the sensor unit carrier 130 moves in the first direction, the inclined surface 1119a of the first acceleration latch 1118 and the inclined surface 1328a of the second acceleration latch 1328 may move while in contact with each other in a frictional state. In this case, the extension arm 1320 may be deformed toward an outward direction. Subsequently, as the sensor unit carrier 130 moves toward the first direction, contact between the inclined surface 1328a of the second acceleration latch 1328 and the inclined surface 1119a of the first acceleration latch 1118 may be released, and the support surface 1119b of the first acceleration latch 1118 and the support surface 1328b of the second acceleration latch 1328 may be switched to a state facing each other.

After the support surface 1119b of the first acceleration latch 1118 and the support surface 1328b of the second acceleration latch 1328 are switched to a state facing each other, insertion of the transcutaneous sensor member 330 into the subcutaneous tissue may be performed. After insertion of the transcutaneous sensor member 330 into the subcutaneous tissue, movement of the sensor unit carrier 130 in a direction opposite to the first direction may be restricted by the facing support surface 1119b of the first acceleration latch 1118 and the support surface 1328b of the second acceleration latch 1328.

In the above, a latch structure having a wedge-shaped cross-section provided with the inclined surfaces 1119a and 1328a and the support surfaces 1119b and 1328b has been described by way of example with reference to the shape of the first acceleration latch 1118 and the second acceleration latch 1328, but the shape of the first acceleration latch 1118 and the second acceleration latch 1328 is not necessarily limited thereto. As long as a structure may prevent arbitrary firing of the applicator 10 in a situation unintended by a user through interaction, may provide sufficient acceleration conditions to the needle body 1402 during subcutaneous tissue insertion of the transcutaneous sensor member 330, and may prevent the sensor unit carrier 130 from moving in the direction opposite to the first direction after the subcutaneous tissue insertion of the transcutaneous sensor member 330, the first acceleration latch 1118 and the second acceleration latch 1328 may be variously modified and applied. The first acceleration latch 1118 and the second acceleration latch 1328 may be means for prevent arbitrary firing of the applicator 10 in an unintended situation by a user through interaction, means for grant sufficient acceleration conditions to the needle body 1402 during the insertion of the transcutaneous sensor member 330 into the subcutaneous tissue, or means for prevent movement of the sensor unit carrier 130 in the direction opposite to the first direction after the insertion of the transcutaneous sensor member 330 into the subcutaneous tissue.

The bridge 1330 may be disposed between the extension arm 1320 and the sensor unit carrier body 1310. The bridge is configured to restrict movement of the sensor unit carrier from an initial position to an insertion position. The bridge is configured to be cut or deformed when pressed by the bridge pressing portion 1116. One end and the other end of the bridge 1330 may be provided to be connected to one side surface of the sensor unit carrier body 1310 and to one surface of the extension arm 1320 facing the sensor unit carrier body 1310, respectively. Alternatively, only one end of the bridge 1330 may be provided to be connected to either one side surface of the sensor unit carrier body 1310 or one surface of the extension arm 1320 facing the sensor carrier body 1310. The bridge 1330 may be disposed in a region overlapping with a needle carrier guide protrusion 1313 with respect to the second direction, and may be disposed at a position between the extension arm connection portion 1322 and the second acceleration latch 1328. The bridge 1330 may be disposed at a position closer to the extension arm connection portion 1322 than to the second acceleration latch 1328.

The bridge 1330 may preferably be formed at a position corresponding to a bridge pressing portion 1116 of the column 1110. The bridge 1330 may have one or more vulnerable portions 1332 formed therein to have a relatively small thickness. In a process in which a user presses the handle housing 120 in the first direction so that the sensor unit carrier 130 moves in the first direction, only when the bridge 1330, which comes into contact with the bridge pressing portion 1116, is cut, the sensor unit carrier 130 may further move to an insertion position.

The vulnerable portion 1332 is a region intended to be cut when the bridge 1330 is pressed by the bridge pressing portion 1116, and it is preferable that the vulnerable portion 1332 is designed to be cut only when a force applied as a user presses the handle housing 120 to insert the transcutaneous sensor member 330 into the subcutaneous tissue is applied to the bridge 1330. Here, typically, the force applied as a user presses the handle housing 120 to insert the transcutaneous sensor member 330 into the subcutaneous tissue does not generally mean a force applied in an assembling and transporting process of the applicator assembly 1, but may mean a level of force applied to the handle housing 120 by a user of the applicator assembly 1 intending insertion of the transcutaneous sensor member 330 into the subcutaneous tissue.

Although a region having a thickness smaller than a thickness of the bridge 1330 has been described as one example of the vulnerable portion 1332, the vulnerable portion 1332 is not necessarily limited to a structure having a relatively small thickness. The vulnerable portion 1332 may mean a region having lower fracture strength than the entire bridge 1330 by applying different materials or the like, or by applying a vulnerable structure. Through interaction between the bridge 1330 and the bridge pressing portion 1116, not only arbitrary firing of the applicator 10 in unintended situations by a user may be prevented, but also sufficient acceleration conditions may be granted to the needle body 1402 during insertion of the transcutaneous sensor member 330 into the subcutaneous tissue.

Although, in the above case where the bridge 1330 is provided on the sensor unit carrier 130 and the bridge pressing portion 1116 is provided on a leading end portion of the partition wall 1112 has been exemplarily described, it may include a case where a bridge structure is provided on the partition wall 1112 and a pressing portion for cutting the bridge structure is provided on the sensor unit carrier 130.

At an end portion of the extension arm 1320, the extension arm pushing portion 1326 having an inclined surface 1326a may be formed to protrude toward the first direction. An extension arm pushing portion 1326 may be provided to have a width relatively smaller than that of an extension arm 1320. The extension arm pushing portion 1326 may be disposed at a central portion side of an end portion of the extension arm 1320, and may also be disposed eccentrically to one side from the central portion side of the end portion of the extension arm 1320. The extension arm pushing portion 1326 may be disposed within a region overlapping with the sensor unit carrier body 1310 with respect to the second direction. A fixing protrusion 1317 formed to protrude from one end of the sensor unit carrier body 1310 may be disposed at a position more protruded than the extension arm pushing portion 1326. When the sensor unit carrier 130 moves along the first direction and is positioned at an insertion position, the extension arm pushing portion 1326 may be positioned so as to press a pushed portion 1165 provided in a transmission unit support hook 1162. By the pushed portion 1165 being pressed by the extension arm pushing portion 1326, the transmission unit support hook 1162 may be deformed outward away from the transmission unit 40, thereby releasing movement restriction of the transmission unit 40 by a catching portion 1164.

The inclined surface 1326a formed in the extension arm pushing portion 1326 and the inclined surface 1165a formed in the pushed portion 1165 are not particularly limited as long as the shape allows the transmission unit support hook 1162 to be deformable toward the outside away from the transmission unit 40 when the pushed portion 1165 is pressed by the extension arm pushing portion 1326. As one example, the extension arm pushing portion 1326 may be provided in a shape in which a cross-section decreases along the first direction, and the inclined surface 1326a of the extension arm pushing portion 1326 may be formed at a position facing an inner side wall of a body housing body portion 1100. At one end of the pushed portion 1165, an inclined surface 1165a corresponding to the inclined surface 1326a of the extension arm pushing portion 1326 may be provided, and the inclined surface 1165a of the pushed portion 1165 may be formed to be inclined in a direction opposite to the inclined surface 1326a of the extension arm pushing portion 1326 with reference to the first direction. It may be preferable that the extension arm pushing portion 1326 is formed at a position capable of pressing the pushed portion 1165 simultaneously with the sensor unit carrier 130 reaching the insertion position or immediately before the sensor unit carrier 130 reaches the insertion position.

At one end portion of the second movement space 1312 adjacent to the handle housing 120, a sensor unit carrier ring portion 1316 to which an elastic member 150 to be described later is fixedly disposed may be provided. The sensor unit carrier ring portion 1316 may be disposed at a position facing a fourth opening 1314, and may be disposed inside a second movement space 1312. Meanwhile, on one surface of the sensor unit carrier body 1310 opposite to the second movement space 1312, a fixing groove 1340 having a shape corresponding to a fixing protrusion portion 1137 may be formed to be recessed. The fixing groove 1340 may preferably be formed to be recessed at a position corresponding to the fixing protrusion portion 1137 when the sensor unit carrier 130 is positioned at an initial position.

### Needle carrier

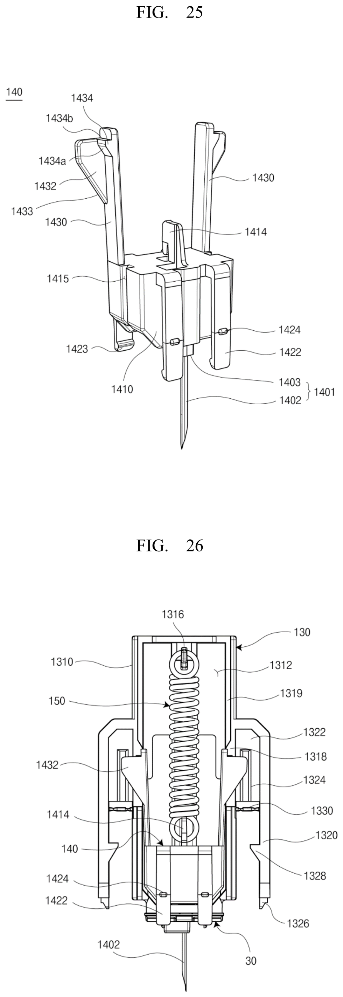

FIG. 24 is a perspective view exemplarily illustrating a coupling relationship of the needle carrier 140, FIG. 25 is a perspective view illustrating an exemplary embodiment of the needle carrier 140, and FIG. 26 is a front view exemplarily illustrating a coupling relationship between the needle carrier 140 and the sensor unit carrier 130.

The needle carrier 140 may be configured to move along the first direction together with the sensor unit carrier 130 and the sensor unit 30 by being provided with the needle 1401 for the insertion of the transcutaneous sensor member 330 into the subcutaneous tissue, and may be configured to move in a direction opposite to the first direction in the second movement space 1312 after the insertion of the transcutaneous sensor member 330 into the subcutaneous tissue to remove the needle body 1402 from the subcutaneous tissue.

The needle carrier 140 may include the needle 1401 and the needle carrier body 1410. The needle carrier body 1410 may fix the needle 1401 and be configured to move together with the needle 1401. The needle 1401 may include the needle body 1402 provided such that one end thereof is inserted into the subcutaneous tissue for the insertion of the transcutaneous sensor member 330 into the subcutaneous tissue, and a needle holder 1403 for fixing the needle body 1402. The needle body 1402 may have an opening portion opened radially outward based on an extension direction of the needle body 1402, and the opening portion may be formed to extend along the first direction. The needle body 1402 may have one side end portion adjacent to the transmission unit accommodation portion 1104 opened. One side end portion of the needle body 1402 adjacent to the transmission unit accommodation portion 1104 may be formed to be inclined.

At one end of the needle carrier body 1410 facing the fourth opening 1314, a needle holder insertion groove 1412 into which the needle holder 1403 is inserted and fixed may be formed to be recessed. By the needle holder 1403 being inserted and fixed in the needle holder insertion groove 1412, the needle 1401 may be fixed to the needle carrier body 1410.

When the needle carrier 140 is positioned at an initial position and an insertion position, the needle body 1402 may be discharged to the exterior through the fourth opening 1314, and when the needle carrier 140 is restored to a retracted position, the needle body 1402 may be introduced through the fourth opening 1314 and disposed in the interior of the second movement space 1312.

On one side surface of the needle carrier body 1410, the needle guide groove 1415 having a shape recessed from one side surface of the needle carrier body 1410 and extending along the first direction may be provided. A needle guide groove 1415 may be provided in a shape extending from a front end of a needle carrier body 1410 toward a rear end to terminate one side surface of the needle carrier body 1410. A leading end portion of the needle carrier guide protrusion 1313 described above may be disposed inside the needle guide groove 1415. By interaction between the needle carrier guide protrusion 1313 and the needle guide groove 1415, movement of the needle carrier 140 in a direction opposite to the first direction may be guided. The needle guide groove 1415 may be formed to be recessed in a pair symmetrically at both side ends of the needle carrier body 1410.

A needle carrier wing body 1430 may be provided in a pair in a shape extending toward a direction opposite to the first direction from both side ends of the needle carrier body 1410. The needle carrier wing body 1430 may be disposed to extend from both side ends of the needle carrier body 1410 in which the needle guide groove 1415 is formed. The needle guide groove 1415 and a needle holder insertion groove 1412 may be respectively disposed on one surface and the other surface opposite to each other of the needle carrier wing body 1430, and may be disposed at positions not overlapping with each other with respect to the first direction. The needle carrier wing body 1430 may be deformed upon application of an external force and may be provided to be restored to an original state after removal of the external force. At a leading end portion of the needle carrier wing body 1430, the needle carrier latch 1434 and the trigger 1432 may be provided. The needle carrier latch 1434 and the trigger 1432 may be provided at positions not overlapping with a needle carrier body 1410 with respect to the first direction. The needle carrier latch 1434 may include a constraint surface 1434b formed in a direction substantially parallel to the second direction, and an inclined surface 1434a inclinedly disposed to become closer to the needle carrier wing body 1430 toward the first direction from one end of the protruded constraint surface 1434b. The constraint surface 1434b and the inclined surface 1434a may be sequentially disposed with respect to the first direction.

The trigger 1432 may be provided in a shape protruding more outward and occupying a larger area than the needle carrier latch 1434. The trigger 1432 may be provided with a trigger inclined portion 1433 so as to have a shape in which a cross-section of the trigger 1432 decreases along the first direction. The trigger inclined portion 1433 may be provided at a position more spaced in the first direction than the inclined surface 1434a, and the trigger inclined portion 1433 and the inclined surface 1434a may be provided at positions not overlapping with respect to the first direction. The trigger 1432 may preferably be disposed at the outside of the second movement space 1312.

Before operation of the applicator assembly 1 or when the needle carrier 140 moves along the first direction, since the constraint surface 1434b of the needle carrier latch 1434 maintains a state of being in contact with the detent constraint surface 1318b of the sensor unit carrier detent 1318, relative movement of the needle carrier 140 with respect to the sensor unit carrier 130 may be restricted. That is, in a state where the constraint surface 1434b of the needle carrier latch 1434 is in contact with the detent constraint surface 1318b of the sensor unit carrier detent 1318, independent movement of the needle carrier 140 is impossible, and the needle carrier 140 may move together only when the sensor unit carrier 130 moves in a state in which the needle carrier 140 is constrained to the sensor unit carrier 130.

When the needle carrier 140 moves to a position adjacent to the insertion position in the insertion process of the transcutaneous sensor member 330 into the subcutaneous tissue, the trigger inclined portion 1433 of the trigger 1432 comes into contact with a leading end portion of the partition wall 1112, so that the needle carrier wing body 1430 may be deformed toward the inside of the second movement space 1312. As the needle carrier wing body 1430 is deformed toward the inside of the second movement space 1312, the contact constraint of the constraint surface 1434b of the needle carrier latch 1434 with the detent constraint surface 1318b of the sensor unit carrier detent 1318 may be released.

By releasing the contact constraint of the constraint surface of the needle carrier latch 1434 from the detent constraint surface 1318b of the sensor unit carrier detent 1318, the needle carrier latch 1434 may ride over the sensor unit carrier detent 1318, and by an elastic force applied from the elastic member 150, the needle carrier 140 may move in a direction opposite to the first direction and reach a retracted position.

To achieve purposes such as preventing pain due to excessive insertion of the human body B of the needle body 1402, and the like, it may be preferable that movement constraint of the sensor unit carrier 130 with respect to the needle carrier 140 be released before the sensor unit carrier 130 reaches the insertion position. In this case, even if the needle carrier 140 moves in the direction opposite to the first direction, the sensor unit carrier 130 maintains a state of pressing the sensor unit 30 toward the first direction, so that the transcutaneous sensor member 330 may be accurately inserted into an intended subcutaneous position by the inherent rigidity of the transcutaneous sensor member 330.

A plurality of grip arms 1422 may be provided in a shape extending along the first direction from other both side ends of the needle carrier body 1410. That is, a plurality of grip arms 1422 may be formed to extend from one surface adjacent to one surface of a needle carrier body 1410 in which a needle guide groove 1415 is formed. A plurality of grip arms 1422 may also be provided in a pair with a symmetrical shape respectively to the needle carrier body 1410. At a leading end portion of the grip arm 1422, a grip portion 1423 having a shape protruding inward may be provided, and a side end portion of the sensor unit housing 310 may be gripped by the grip portion 1423. The grip arm 1422 may be formed in a shape extending to a region exceeding a needle holder insertion groove 1412, and the grip portion 1423 may be disposed at a position not overlapping with the needle carrier body 1410 with respect to the second direction. A grip arm protrusion portion 1424 may be formed to protrude from one surface of the grip arm 1422 facing a partition wall of a column 1110. The grip arm protrusion portion 1424 may be provided to each of the grip arms 1422, and may be provided in a region overlapping with the needle carrier body 1410 with respect to the second direction.

When the needle carrier 140 is positioned at an initial position, the grip arm protrusion portion 1424 may be disposed in an interior of a grip arm guide groove 1120 before passing through a first step inclined surface 1123, or may maintain a state of being closely pressed by a step portion 1121 after passing through the first step inclined surface 1123. In the insertion process of the transcutaneous sensor member 330 into the subcutaneous tissue, the grip arm protrusion portion 1424 may pass through the second step inclined surface 1124, and the close-contact pressing of the grip arm protrusion portion 1424 by the step portion 1121 may be released, and in a state where each of the grip arms 1422 is somewhat spread outward, the sensor unit 30 may be delivered to the transmission unit 40.

At one end of the needle carrier body 1410 facing the sensor unit carrier ring portion 1316, a needle carrier ring portion 1414 may be provided, and the other end portion of the elastic member 150, of which one end is connected to the sensor unit carrier ring portion 1316, may be connected to the needle carrier ring portion 1414. The needle carrier ring portion 1414 may be formed to protrude at a central portion of the needle carrier body 1410, and may be formed to protrude with a protrusion height lower than that of the needle carrier wing body 1430.

The elastic member 150 may be applied without limitation as long as it is a means capable of providing a driving force when the needle carrier 140 moves in a direction opposite to the first direction, but a tension spring may be preferably applied.

### Cap

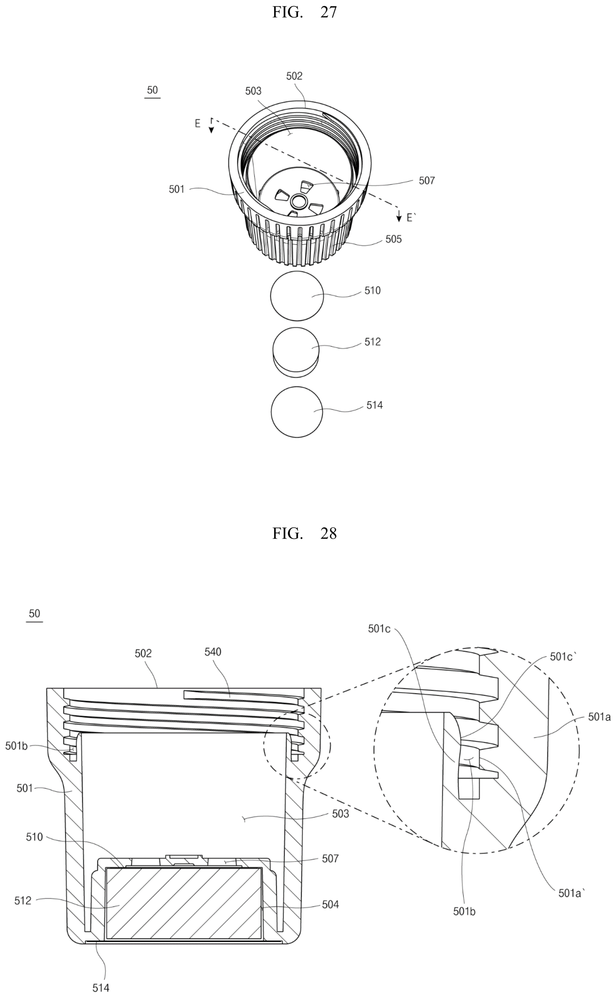

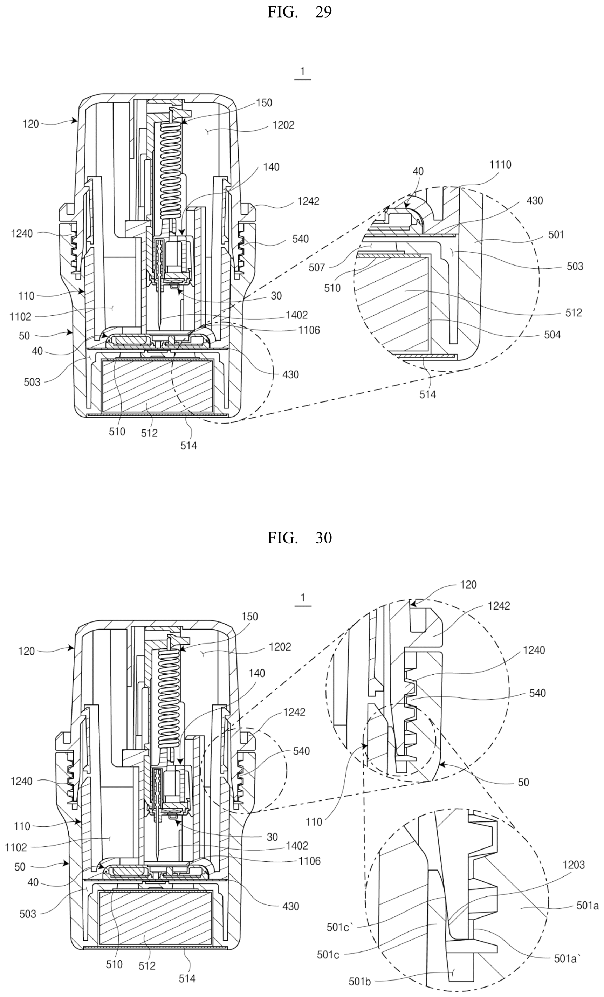

FIG. 27 is an exploded perspective view illustrating an exemplary embodiment of the cap 50, FIG. 28 is a cross-sectional view of the cap 50 cut along the E-E' direction in FIG. 27. FIGS. 29 to 31 are partially enlarged cross-sectional views illustrating an exemplary embodiment of the applicator assembly 1 to which the cap 50 is applied.

The cap 50 may be fixedly disposed so as to be releasable at one end of the applicator 10. Since the applicator assembly 1 includes the cap 50, it may effectively prevent unintended arbitrary firing of the applicator 10 or introduction of external contaminants or moisture into the interior of the applicator assembly 1, which may occur in manufacturing, distribution, storage, and use processes of the applicator assembly 1.

The cap 50 may include a cap housing 501 forming an external shape of the cap 50. In an interior of the cap housing 501, an accommodation space 503 communicating with the exterior through a fifth opening 502 formed at one end of the cap housing 501 may be formed. On an inner surface 501a' at one end of the cap housing 501 where the fifth opening 502 is formed, the screw threaded portion 540 having a shape corresponding to the screw threaded portion 1240 formed on the handle housing 120 may be provided. The cap housing 501 may be means for protecting and dehumidifying the interior of the applicator 10.

As the screw threaded portion 1240 formed on the handle housing 120 is screw-coupled to the screw threaded portion 540 formed on the cap housing 501, the cap 50 may be separably screw-coupled to one end of the handle housing 120.

At an end portion side of a cap housing 501 in which a fifth opening 502 is formed, a first coupling portion 501a and a second coupling portion 501c branched and formed may be provided. On an inner surface 501a' of the first coupling portion 501a, a screw threaded portion 540 may be formed. The second coupling portion 501c may be disposed substantially in parallel with the first coupling portion 501a at an inner side of the first coupling portion 501a. The second coupling portion 501c may be disposed such that its end portion is positioned inside an accommodation space 503 compared with an end portion of the first coupling portion 501a.

On one surface of the second coupling portion 501c facing the inner surface 501a' of the first coupling portion 501a, a protrusion portion 501c' protruding in a rounded shape toward the first coupling portion 501a may be provided to extend along a circumferential direction of the second coupling portion 501c. The second coupling portion 501c may be manufactured of the same material as the first coupling portion 501a, but may also be manufactured of a material superior in elasticity or flexibility compared with the first coupling portion 501a.

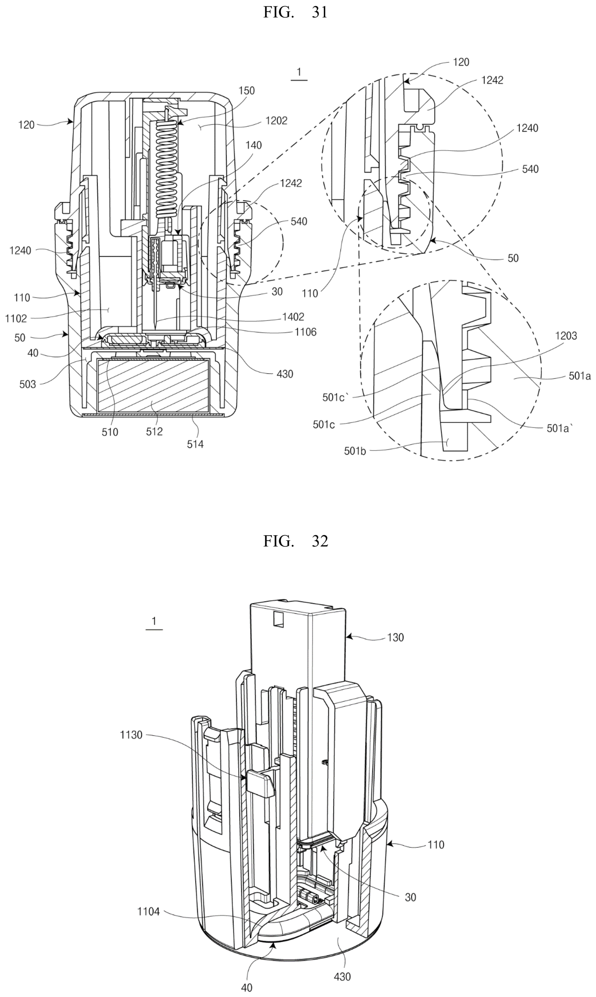

As illustrated in FIG. 30 and FIG. 31, when a cap 50 is coupled to a handle housing 120, an end portion of the handle housing 120 may be disposed in a coupling space 501b between the first coupling portion 501a and the second coupling portion 501c. In this case, the protrusion portion 501c' may be closely attached to a close contact surface 1203 inside the handle housing 120, and a coupling region between the handle housing 120 and the cap 50 may be sealed.

On an outer surface of the cap housing 501, a plurality of grip grooves 505 may be formed to be recessed in order to facilitate the work by a worker or user or the convenience of use. The plurality of grip grooves 505 may be formed in a shape extending along the first direction, and each of the grip grooves 505 may be disposed spaced apart from each other at regular intervals. The number and shape of the grip grooves 505 are not limited to the matters illustrated in the drawings, and the number and shape of the grip grooves 505 may be variously modified and applied.

Meanwhile, although the drawings illustrate the cap housing 501 in a cup shape, the shape of the cap housing 501 of the present invention is not necessarily limited thereto. As long as the shape allows protection and dehumidification of an interior of an applicator 10, the shape of a cap housing 501 may be variously modified and applied. However, since one end of the cap housing 501 in which the screw threaded portion 540 is formed is intended to be screw-coupled to the screw threaded portion 1240 formed on the handle housing 120, it may be preferable that the end portion of the cap housing 501 in which the screw threaded portion 540 is formed be provided in a cylindrical shape corresponding to the end portion of the handle housing 120.

At the other closed end portion of the cap housing 501 facing the fifth opening 502, an accommodation groove 504 having a shape recessed from the outside toward the accommodation space 503 may be provided. At the other end portion of the cap housing 501 forming the accommodation groove 504, a plurality of ventilation holes 507 communicating the accommodation groove 504 with the accommodation space 503 may be formed to penetrate. In the accommodation groove 504, a first sealing member 510, a dehumidifier 512, and a second sealing member 514 may be sequentially disposed from one side of the ventilation holes 507 toward the outside.

The first sealing member 510 may preferably be manufactured using a material that allows air to pass through but does not allow moisture to pass through, and as a non-limiting example, Tyvek material of DuPont may be used. By means of the first sealing member 510, introduction of moisture into the accommodation space 503 may be effectively prevented.

The dehumidifier 512 may preferably be manufactured using a material having a dehumidifying function applied to an electronic device or a medical device. By means of the dehumidifier 512, moisture introduced into the accommodation space 503 may be removed.

The second sealing member 514 may be disposed so as to seal the accommodation groove 504 from the exterior, and may be manufactured by applying a material that prevents the passage of moisture and external contamination sources. As a non-limiting example, the second sealing member 514 may be manufactured using an aluminum packaging sheet.

Since the first sealing member 510, the dehumidifier 512, and the second sealing member 514 are sequentially disposed in the accommodation groove 504 communicating with the accommodation space 503 through the ventilation holes 507, not only can moisture introduced into the interior of the accommodation space 503 be effectively removed, but also introduction of contaminants or moisture from the exterior into the interior of the accommodation space 503 can be effectively prevented.

During manufacture of an applicator assembly 1, the handle housing 120 and the cap 50 may be sterilized in a state where they are coupled after attaching a first sealing member 510 to an accommodation groove 504, and then the interior of the applicator assembly 1 may be sterilized. After sterilization, a dehumidifier 512 may be disposed in the accommodation groove 504, and a second sealing member 514 may be fused to an end portion of the accommodation groove 504 in which the dehumidifier 512 is disposed, so that sterilization and dehumidification processing of the interior of the applicator assembly 1 may be sequentially performed. Unlike in a case where sterilization and dehumidification processing is performed after disposing the applicator assembly 1 in separate packaging material or a packaging container, sterilization and dehumidification processing may be performed in an assembly process of the applicator assembly 1, thereby simplifying a manufacturing process of the applicator assembly 1.

Meanwhile, before subcutaneous tissue insertion of a sensor member 330, a sensor unit 30 and a transmission unit 40 may maintain a separated state, and since the sensor unit 30 and a needle 1401 maintain a state disposed in an interior of the applicator 10, an end portion of the sensor member 330 or the needle 1401 may not extend to the accommodation groove 504 of the cap 50. Accordingly, the structure of the cap 50 may be simplified by simplification of a sterilization and dehumidification structure, and as the structure of the cap 50 is simplified, the applicator assembly 1 coupled with the cap 50 may have a compact size.

As illustrated in FIG. 31, sealing members 508a and 508b, which are disposed upright in a ring shape, may be provided in a pair at a leading end portion of the cap housing 501, and a caulking member 1243 may be protrudingly formed on one surface of the locking protrusion portion 1242 corresponding to the leading end portion of the cap housing 501. When the screw threaded portion 540 formed in the cap housing 501 and the screw threaded portion 1240 formed in the handle housing 120 are screw-coupled to each other such that the cap 50 is coupled to one end of the handle housing 120, the sealing members 508a and 508b may closely contact and press the caulking member 1243 to effectively prevent introduction of external contaminants or moisture into the space between the handle housing 120 and the cap 50.

The caulking member 1243 and the sealing members 508a and 508b may include a case where they are integrally injection-molded together with the handle housing 120 and the cap housing 501, or a case where they are separately manufactured using a material having inherent elasticity such as rubber or silicone. Even in a case where a caulking member 1243 and sealing members 508a and 508b are integrally injection-molded together with a handle housing 120 and a cap housing 501, the caulking member 1243 and the sealing members 508a and 508b may be injection-molded of materials different from each other distinguished from the handle housing 120 and the cap housing 501.

Hereinafter, with reference to FIGS. 32 to 49, the assembly and operation processes of the applicator 10 and the applicator assembly 1 will be described in more detail.

### Exemplary embodiment of action of fixing portion

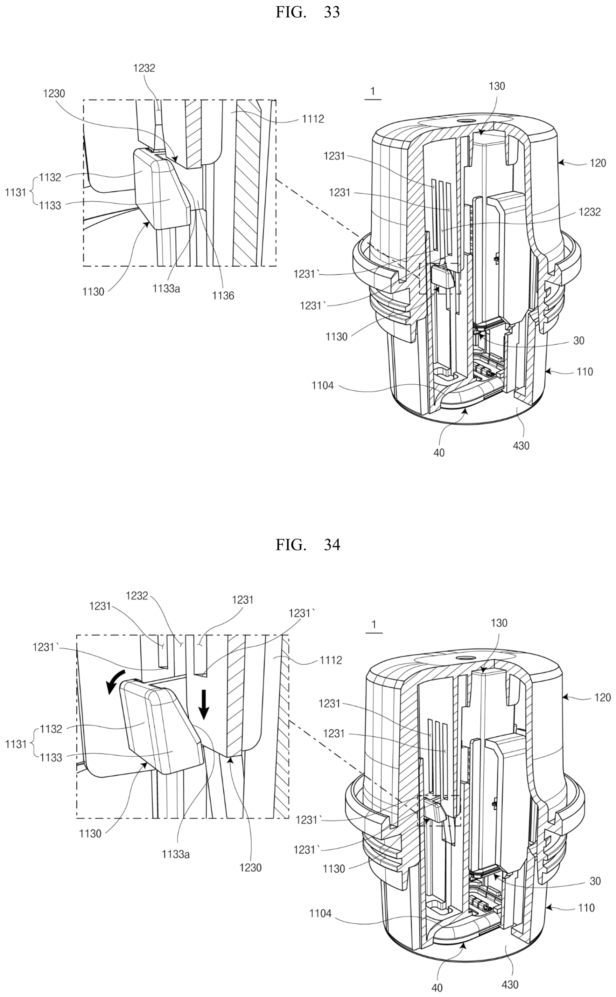

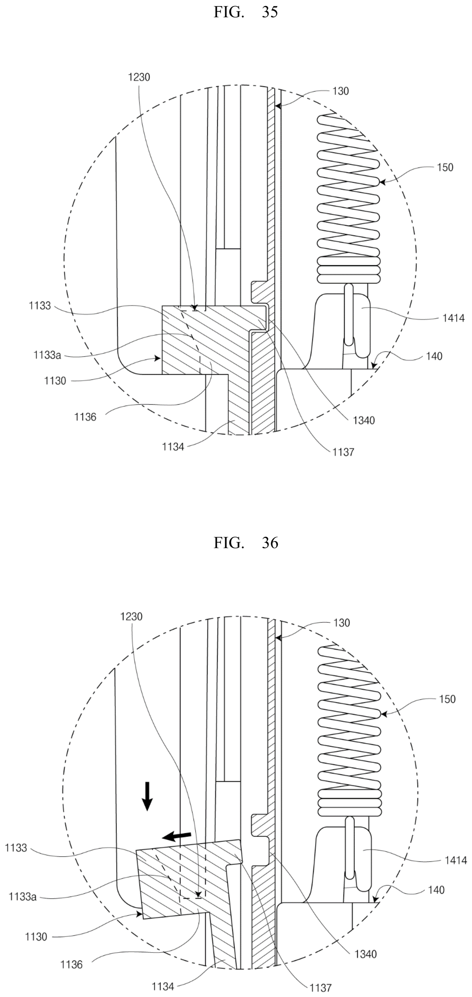

FIG. 32 is a partial cross-sectional view illustrating an exemplary embodiment in a state where the sensor unit carrier 130 is coupled to the body housing 110 during an assembly process of the applicator assembly 1, and FIGS. 33 and 34 are partial cross-sectional views illustrating an exemplary embodiment of a process of coupling the handle housing 120 to the body housing 110 to which the sensor unit carrier 130 is coupled during the assembly process of the applicator assembly 1. FIG. 35 is an enlarged cross-sectional view illustrating an exemplary embodiment in a state where a leading end portion of the fixing protrusion portion 1137 is inserted into the temporary fixing groove 1340 during the assembly process of the applicator assembly 1, and FIG. 36 is an enlarged cross-sectional view illustrating an exemplary embodiment in a state where the leading end portion of the fixing protrusion portion 1137 is discharged from the temporary fixing groove 1340 during the assembly process of the applicator assembly 1.

As illustrated in FIGS. 32 and 35, in a process of assembling the body housing 110 and the sensor unit carrier 130, the sensor unit carrier body 1310 may be fitted into the column 1110 forming the first movement space 1111. In the process of fitting the sensor unit carrier body 1310 into the column 1110, the fixing protrusion portion 1137 may come into contact with one surface of the sensor unit carrier body 1310, and the support 1134 may maintain a state deformed outward. As the sensor unit carrier body 1310 moves along the first direction, the support 1134 may maintain the state deformed outward, and as the fixing groove 1340 formed in the sensor unit carrier body 1310 reaches a position corresponding to the fixing protrusion portion 1137, the support 1134 may be restored to an original state from the deformed state. That is, as the leading end portion of the fixing protrusion portion 1137 is introduced into the fixing groove 1340, the pressing movement portion 1131 may move toward the sensor unit carrier body 1310, and the support 1134 may also be restored from deformation.

As the leading end portion of the fixing protrusion portion 1137 is introduced into the fixing groove 1340, movement of the sensor unit carrier 130 in the first direction may be restricted. In the assembly process of the applicator assembly 1, a worker may recognize, through a sound generated by parts hitting each other or a sensation transmitted to fingertips, that the leading end portion of the fixing protrusion portion 1137 has been normally introduced into the fixing groove 1340.

The fixing portion 1130 may be provided in plurality, or a plurality of fixing protrusion portions 1137 may be provided in one fixing portion 1130, and the fixing groove 1340 may be formed to be recessed on one surface of the sensor unit carrier body 1310 in a shape and number corresponding thereto. In order to reduce friction in a process in which at least one end of the fixing protrusion portion 1137 is introduced into and discharged from the fixing groove 1340, an inclined surface may be provided on the fixing groove 1340 and/or the fixing protrusion portion 1137. The fixing groove 1340 may include not only a case of being formed to be recessed on one surface of the sensor unit carrier body 1310, but also a case of being formed to penetrate one surface of the sensor unit carrier body 1310.

Subsequently, the worker may perform a work of coupling the handle housing 120 to the body housing 110 to which the sensor unit carrier 130 is coupled. When a handle housing 120 is coupled to a body housing 110 to which a sensor unit carrier 130 is coupled, the sensor unit carrier 130 may be pressed by the handle housing 120 into which the sensor unit carrier 130 enters for assembly, so that arbitrary firing may occur or the sensor unit carrier 130 may deviate from an initial position.

When assembly of the handle housing 120 is performed in a state in which a leading end portion of a fixing protrusion portion 1137 is introduced into a fixing groove 1340, arbitrary firing of an applicator 10 or positional deviation of the sensor unit carrier 130 may be effectively prevented.

As illustrated in FIGS. 33, 34, and 36, in the process of coupling the handle housing 120 to the body housing 110 to which the sensor unit carrier 130 is coupled, the leading end portion of the push arm 1230 provided in the handle housing 120 moves to a position in contact with the pressing movement inclined surface 1133a of the pressing movement wing portion 1133. As the assembly of the handle housing 120 proceeds, the leading end portion of the push arm 1230, in a state of being in contact with the pressing movement inclined surface 1133a, move in the first direction to press the pressing movement wing portion 1133, and the support 1134 is deformed outward, whereby the pressing movement portion 1131 is pushed in a direction away from the sensor unit carrier body 1310. As the pressing movement portion 1131 is pushed in the direction away from the sensor unit carrier body 1310, the leading end portion of the fixing protrusion portion 1137 may be discharged from the fixing groove 1340. As the leading end portion of the fixing protrusion portion 1137 is discharged from the fixing groove 1340, arbitrary movement restriction of the sensor unit carrier 130 by the fixing portion 1130 is released, and the sensor unit carrier 130 may maintain a state of being able to move irrespective of the fixing portion 1130.

Although FIG. 34 illustrates a state in which a leading end of the sensor unit carrier 130 completely comes into contact with an inner surface of the handle housing 120 after completion of assembly of the applicator 10, it may include a case where, as illustrated in FIG. 33, the leading end of the sensor unit carrier 130 maintains a state of being spaced apart from the inner surface of the handle housing 120 at a predetermined interval even after completion of assembly of the applicator 10. In this case, during a process in which a user presses the handle housing 120 so that the handle housing 120 moves from the first position to the second position, a spacing distance between the leading end of the sensor unit carrier 130 and the handle housing 120 may be maintained or narrowed, and in an operation process of the applicator assembly 1, when excessive pressure is applied to the handle housing 120, a buffer may be provided against delivery of the corresponding pressure as it is to the transcutaneous sensor member 330.

### Exemplary embodiment of operation of needle carrier

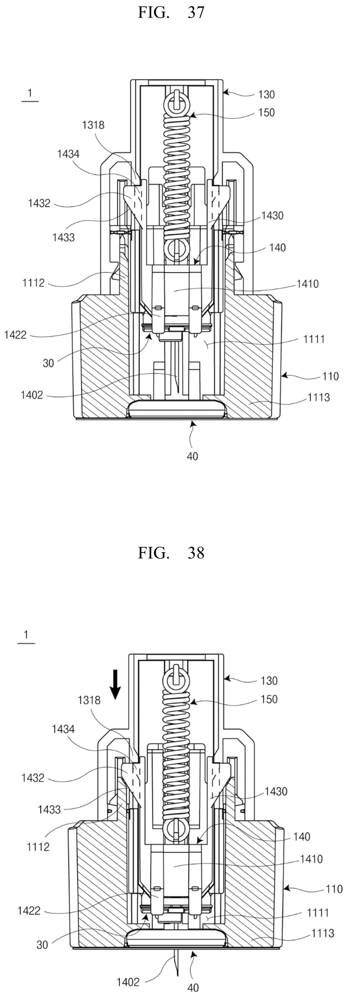

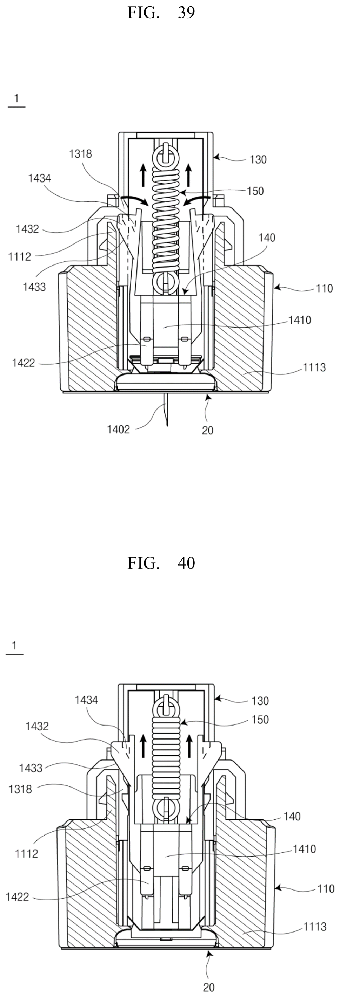

FIGS. 37 to 39 are partial cross-sectional views sequentially illustrating an exemplary embodiment of a state in which the sensor unit carrier 130 moves from an initial position to an insertion position during an operation process of the applicator assembly 1, and FIG. 40 is a partial cross-sectional view illustrating an exemplary embodiment of a state in which the needle carrier 140 moves to a retracted position during the operation process of the applicator assembly 1.

As illustrated in FIG. 37, in a process in which the sensor unit carrier 130 moves from the initial position toward the insertion position, since the constraint surface 1434b of the needle carrier latch 1434 maintains a state of being in contact with the detent constraint surface 1318b of the sensor unit carrier detent 1318, the needle carrier 140 may maintain a state constrained to the sensor unit carrier 130 and may move along the first direction together with the sensor unit carrier 130.

As illustrated in FIG. 38, when the sensor unit carrier 130 reaches an insertion position or a position adjacent to the insertion position, the trigger inclined portion 1433 of the trigger 1432 may contact a leading end portion of the partition wall 1112. Meanwhile, as illustrated in FIG. 39, as the sensor unit carrier 130 continuously moves in the first direction, the needle carrier wing body 1430 is deformed toward the inside of the second movement space 1312, and accordingly, the constraint surface 1434b of the needle carrier latch 1434 and the detent constraint surface 1318b of the sensor unit carrier detent 1318 may deviate from a position of being in mutual contact. Therefore, the needle carrier latch 1434 becomes released from the constraint of the sensor unit carrier detent 1318, and the needle carrier 140 may relatively move with respect to the sensor unit carrier 130.

Meanwhile, as illustrated in FIGS. 38 and 39, the release of the movement constraint of the needle carrier 140 by the sensor unit carrier 130 may be performed before the sensor unit carrier 130 reaches the insertion position, and accordingly, side effects due to excessive human body B insertion of the needle body 1402 can be effectively prevented. That is, release of a movement constraint of a needle carrier 140 by a sensor unit carrier 130 may be performed not only in a state in which a leading end portion of a grip arm 1422 contacts one end of a transmission unit 40, but also in a state in which the leading end portion of the grip arm 1422 is somewhat spaced apart from one end of the transmission unit 40. When the movement constraint of the needle carrier 140 is released by the sensor unit carrier 130, the needle carrier 140 may move in a direction opposite to the first direction by an elastic force of an elastic member 150.

When the movement constraint of the needle carrier 140 is released by the sensor unit carrier 130 in a state in which the leading end portion of the grip arm 1422 is somewhat spaced apart from one end of the transmission unit 40, the risk of failure occurrence or coupling defect that may occur as the grip arm 1422 collides or contacts the transmission unit 40 may be effectively reduced.

As illustrated in FIG. 40, after a constraint of a needle carrier latch 1434 by a sensor unit carrier detent 1318 is released, the needle carrier 140 may move relative to the sensor unit carrier 130. The needle carrier 140 may move in a direction opposite to the first direction by a driving force applied from the elastic member 150, and may reach a retracted position.

When the needle carrier 140 reaches the retracted position, since the leading end portion of the needle body 1402 is disposed so as to be entirely accommodated in the first movement space 1111, a state in which the needle body 1402 is no longer exposed or protruded to the exterior of the applicator 10 may be maintained.

### Exemplary embodiment of action of bridge

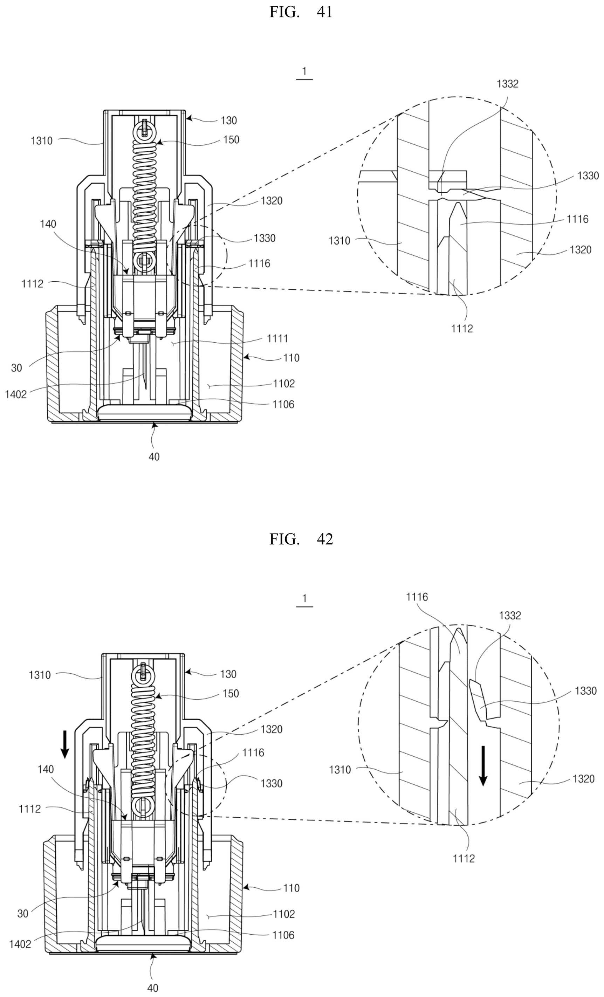

FIG. 41 is a partial cross-sectional view illustrating an exemplary embodiment of a state before the bridge 1330 is cut during an operation process of the applicator assembly 1, and FIG. 42 is a partial cross-sectional view illustrating an exemplary embodiment of a state in which the bridge 1330 is cut during the operation process of the applicator assembly 1.

FIG. 41 is a view for describing a positional relationship between the bridge 1330 and the bridge pressing portion 1116 in case where the sensor unit carrier 130 is positioned at an initial position, but as illustrated in FIG. 41, it may include not only a case where the bridge 1330 is disposed at a position spaced apart from the bridge pressing portion 1116 at a predetermined interval at the initial position, but also a case where a leading end portion of the bridge pressing portion 1116 maintains a state of coming into contact with the bridge 1330 at the initial position.

When the handle housing 120 is pressed and moved in the first direction by a user, the sensor unit carrier 130 also may move along the first direction together with the handle housing 120. Only when a force greater than fracture strength of the bridge 1330 designed in a manufacturing process is applied to the handle housing 120, the bridge 1330 may be fractured as illustrated in FIG. 42, and only when the bridge 1330 is fractured, normal firing and subcutaneous insertion of the transcutaneous sensor member 330 may be performed.

Although FIG. 42 exemplarily describes that fracture of the bridge 1330 occurs at the vulnerable portion 1332, it may include a case where fracture of the bridge 1330 occurs at a region other than the vulnerable portion 1332.

A vulnerable portion 1332 may be provided in plurality, and a bridge pressing portion 1116 may be disposed to face a region between the vulnerable portion 1332 or the plurality of vulnerable portions 1332. Among the plurality of vulnerable portions 1332, the vulnerable portion relatively adjacent to the sensor unit carrier body 1310 may be fractured, and the vulnerable portion relatively farther away from the sensor unit carrier body 1310 may be partially fractured and bent. The bridge pressing portion 1116 may be disposed to face a region relatively adjacent to the sensor unit carrier body 1310 among the regions between the plurality of vulnerable portions 1332.

The plurality of vulnerable portions 1332 may have the same thickness, or may have different thicknesses. The vulnerable portion 1332 relatively adjacent to the sensor unit carrier body 1310 or the vulnerable portion 1332 relatively adjacent to a portion contacting the bridge pressing portion 1116 may have a thickness thinner than other vulnerable portions 1332.

### Exemplary embodiment of interaction between first movement restriction portion and second movement restriction portion

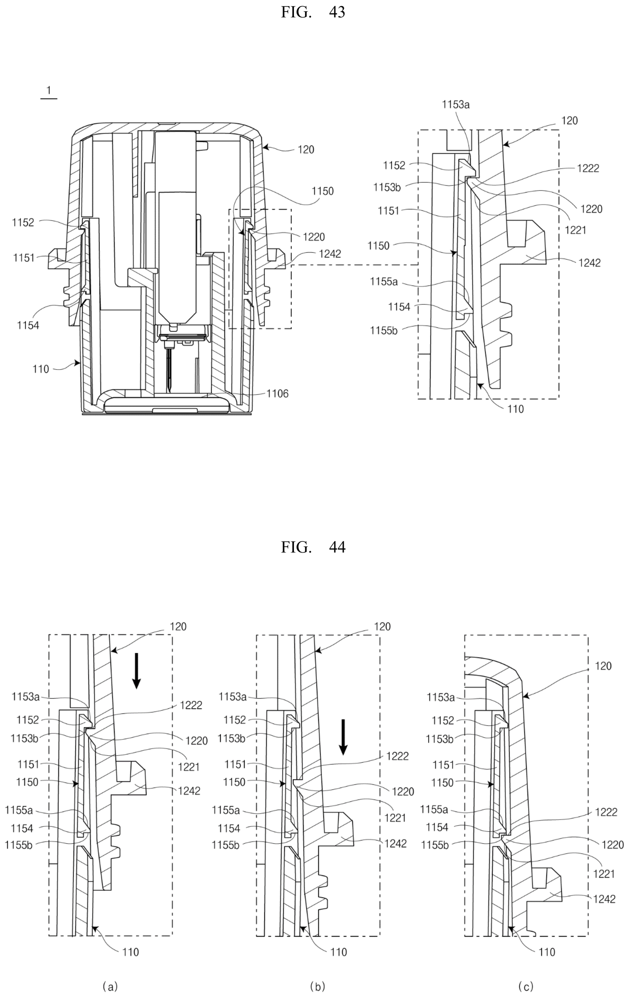

FIG. 43 is a cross-sectional view and a partial enlarged cross-sectional view for explaining an exemplary embodiment regarding a positional relationship between the first movement restriction portion 1220 and the second movement restriction portion 1150 in a state before operation of the applicator assembly 1, and FIGS. 44(a) to 41(c) are sequentially illustrated partial enlarged cross-sectional views for explaining an exemplary embodiment regarding a positional relationship between the first movement restriction portion 1220 and the second movement restriction portion 1150 during an operation process of the applicator assembly 1.

As illustrated in FIG. 43 and FIG. 44(a), in a state before operation of the applicator assembly 1, the support surface 1222 of the first movement restriction portion 1220 and the first support surface 1153b of the first movement restriction latch 1152 may be disposed to face each other. Accordingly, even if a force in a direction opposite to the first direction is applied to a handle housing 120, since a support surface 1222 of a first movement restriction portion 1220 and a first support surface 1153b of a first movement restriction latch 1152 contact and support each other, the handle housing 120 cannot move further in the direction opposite to the first direction. Accordingly, it is possible to effectively prevent the handle housing 120 from being arbitrarily deviated from the body housing 110.

Meanwhile, as illustrated in (b) and (c) of FIG. 44, in a firing process of an applicator assembly 1, the first movement restriction portion 1220 may move toward a second movement restriction latch 1154.

As the handle housing 120 moves in the first direction, an inclined surface 1221 of the first movement restriction portion 1220 moves to a position adjacent to a second inclined surface 1155a of the second movement restriction latch 1154. Subsequently, in a state in which the inclined surface 1221 of the first movement restriction portion 1220 and the second inclined surface 1155a of the second movement restriction latch 1154 contact each other, the handle housing 120 further moves in the first direction. Another end of a movement restriction body 1151 is deformed toward a second interior space 1102, so that the first movement restriction portion 1220 may move to pass through the second movement restriction latch 1154.

In the above, a case in which the other end of the movement restriction body 1151 is deformed toward the second interior space 1102 in a moving process of the handle housing 120 has been described by way of example, but depending on a material or thickness of the handle housing 120 and a body connection portion 1157, a subject that is deformed and moved may be variously modified and applied.

After the first movement restriction portion 1220 passes through the second movement restriction latch 1154, the movement restriction body 1151 is restored to a state before deformation, and the support surface 1222 of the first movement restriction portion 1220 and the second support surface 1155b of the second movement restriction latch 1154 are placed in a state facing each other. Since the movement of the handle housing 120 in a direction opposite to the first direction is restricted by the interaction between the support surface 1222 of the first movement restriction portion 1220 and the second support surface 1155b of the second movement restriction latch 1154, reuse of the applicator 10 after firing of the transcutaneous sensor member 330 can be strictly restricted.

### Exemplary embodiment of action of grip arm protrusion portion

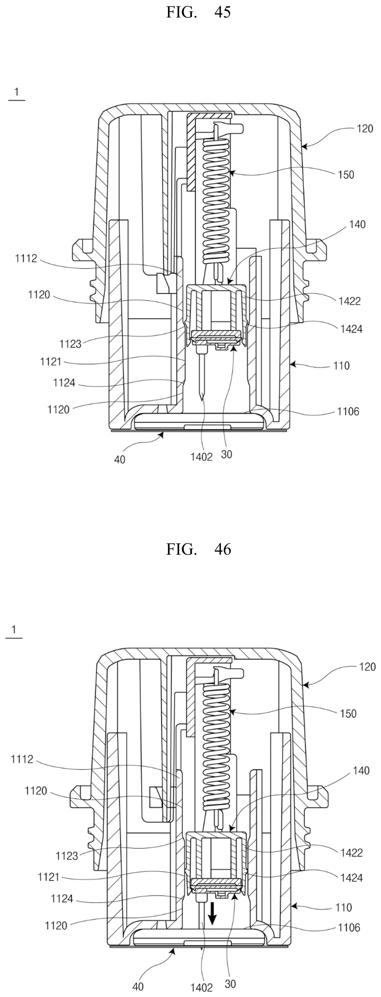

FIGS. 45 to 47 are sequentially illustrated cross-sectional views for explaining an exemplary embodiment of pressing and pressing release of the grip arm protrusion portion 1424 during an operation process of the applicator assembly 1.

FIG. 45 is a view illustrating a state before operation of the applicator assembly 1, and the grip arm protrusion portion 1424 may be disposed in a state of not having passed through the first step inclined surface 1123. That is, since the grip arm protrusion portion 1424 maintains a state of not being pressed by the step portion 1121, the grip arm 1422 may grip the sensor unit 30 in a somewhat loose state.

Meanwhile, unlike FIG. 45, the grip arm protrusion portion 1424 may be disposed in a state of having passed through the first step inclined surface 1123 in the state before operation of the applicator assembly 1, and in this case, the grip arm protrusion portion 1424 is pressed by the step portion 1121 so that the grip arm 1422 may maintain a state of gripping the sensor unit 30 firmly.

When the handle housing 120 is pressed in the first direction by a user, as illustrated in FIG. 46, a grip arm protrusion portion 1424 may maintain a state pressed against a step portion 1121 and may move in the first direction. In this process, a sensor unit 30 may move toward a transmission unit 40 while maintaining a state firmly gripped by a grip arm 1422.

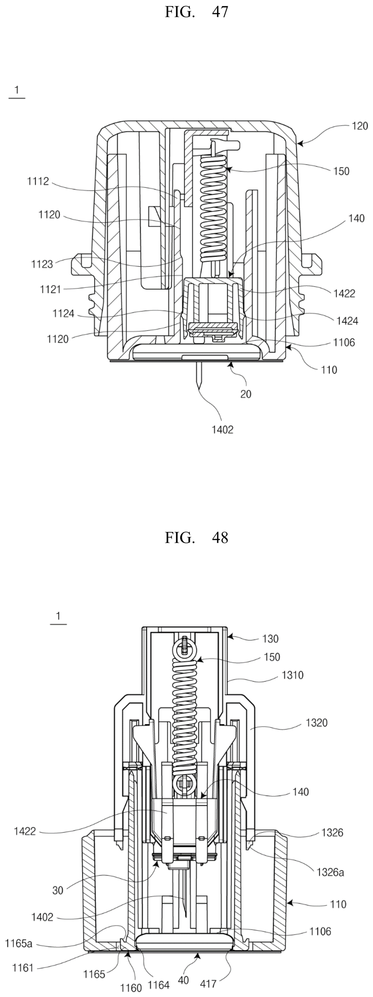

Subsequently, as illustrated in FIG. 47, the grip arm protrusion portion 1424 may pass through a second step inclined surface 1124, and pressing of the grip arm protrusion portion 1424 by the step portion 1121 may be released. The grip arm 1422 may grip the sensor unit 30 in a somewhat loosened state and may deliver the sensor unit 30 to the transmission unit 40. Although the sensor unit 30 may be delivered to the transmission unit 40 simultaneously with the grip arm protrusion portion 1424 passing through the second step inclined surface 1124, it may be more preferable that the sensor unit 30 is delivered to the transmission unit 40 after the grip arm protrusion portion 1424 has passed through the second step inclined surface 1124.

Although release of a constraint of a needle carrier latch 1434 by a sensor unit carrier detent 1318 may be performed simultaneously with the grip arm protrusion portion 1424 passing through the second step inclined surface 1124, with reference to FIG. 39, in terms of accurate delivery of the sensor unit 30, it may be more preferable that release of the constraint of the needle carrier latch 1434 by the sensor unit carrier detent 1318 is performed immediately after the grip arm protrusion portion 1424 has passed through the second step inclined surface 1124.

In the insertion process of the transcutaneous sensor member 330 into the subcutaneous tissue, since the sensor unit 30 continuously receives the force in the first direction by the sensor unit housing 310, even if the grip arm 1422 grips the sensor unit 30 in a somewhat loose state to deliver the sensor unit 30 to the transmission unit 40, the sensor unit 30 may be accurately delivered to the seating groove 412. Since the grip arm 1422 grips the sensor unit 30 in a somewhat loose state to deliver the sensor unit 30 to the seating groove 412 of the transmission unit 40, an operational error that occurs in which the sensor unit 30 is brought along with the needle carrier 140, in a process in which the needle carrier 140 returns to a retracted position, can be effectively prevented.

### Exemplary embodiment of operation of transmission unit support portion

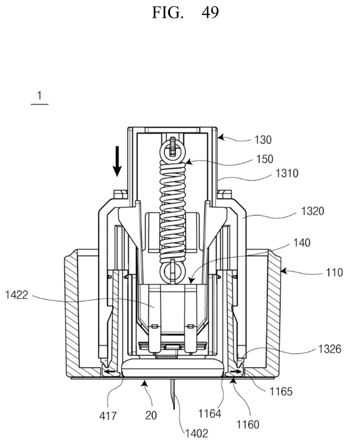

FIGS. 48 and 49 are sequentially illustrated cross-sectional views for explaining an exemplary embodiment of movement constraint and movement constraint release of the transmission unit 40 by the transmission unit support portion 1160 during an operation process of the applicator assembly 1.

As illustrated in FIG. 48, in a state before operation of the applicator assembly 1, a leading end portion of the catching portion 1164 provided in the transmission unit support hook 1162 may be disposed in an interior of the transmission unit housing groove 417 of the transmission unit housing 410 so as to effectively prevent the transmission unit 40 from being arbitrarily deviated from the transmission unit accommodation portion 1104. In this case, the sensor unit carrier 130 is positioned at an initial position, and an extension arm pushing portion 1326 provided to the sensor unit carrier 130 positioned at the initial position may maintain a state spaced apart from a pushed portion 1165 of a transmission unit support hook 1162.

Since the transmission unit 40 is prevented by the transmission unit support hook 1162 from being arbitrarily deviated from the transmission unit accommodation portion 1104 before operation of the applicator assembly 1 or in the insertion process of the transcutaneous sensor member 330 into the subcutaneous tissue, the economic efficiency and operational accuracy of the applicator assembly 1 can be effectively improved.

Meanwhile, as illustrated in FIG. 49, when the applicator assembly 1 operates and the sensor unit carrier 130 moves to an insertion position, the extension arm pushing portion 1326 presses the inclined surface 1165a formed on the pushed portion 1165 so that the transmission unit support hook 1162 moves while being deformed in a direction away from the transmission unit 40, and accordingly, the leading end portion of the catching portion 1164 may be deviated from the transmission unit housing groove 417, thereby releasing the movement constraint of the transmission unit 40 by the transmission unit support hook 1162. That is, when the sensor unit carrier 130 is positioned at an initial position, arbitrary deviation of a transmission unit 40 is restricted by a transmission unit support portion 1160 positioned at a movement constraint position, and as the sensor unit carrier 130 switches and moves to an insertion position, the transmission unit support portion 1160 may switch and move to a movement constraint release position, thereby releasing restriction of arbitrary deviation of the transmission unit 40 by the transmission unit support portion 1160.

The transmission unit support portion 1160, through interaction with the extension arm pushing portion 1326, releases the movement constraint of the transmission unit 40 by the transmission unit support hook 1162 in the insertion process of the transcutaneous sensor member 330 into the subcutaneous tissue, thereby effectively preventing a phenomenon in which the wearable unit 20 is brought along with the applicator 10 in a process of separating the wearable unit 20 from the applicator 10 after completion of the insertion of the transcutaneous sensor member 330 into the subcutaneous tissue.

Meanwhile, the applicator assembly 1 may include a case in which a pushed portion 1165 and an extension arm pushing portion 1326 are not provided. In a state before operation of the applicator assembly 1, at least one end of a leading end portion of a catching portion 1164 may be disposed in an interior of a transmission unit housing groove 417, thereby preventing a transmission unit 40 from arbitrarily deviating from a transmission unit accommodation portion 1104.

In a process of separating the applicator 10 from skin after subcutaneous tissue insertion of a transcutaneous sensor member 330, the transmission unit support portion 1160 may be moved in a direction away from the transmission unit 40 by an adhesive force of a first adhesive surface.

Although the present invention has been described in detail through the embodiments above, other forms of embodiments are also possible. Therefore, the technical teachings and scope of the claims described below are not limited to the embodiments.

### [Description of Reference Numerals]

| | | | |
|---|---|---|---|
| 1: | Applicator assembly | 5: | External terminal |
| 10: | Applicator | 20: | Wearable unit |
| 30: | Sensor unit | 40: | Transmission unit |
| 50: | Cap | 110: | Body housing |
| 120: | Handle housing | 130: | Sensor unit carrier |
| 140: | Needle carrier | 150: | Elastic member |

## Claims

1. An applicator, comprising:
a body housing in which an accommodation portion capable of accommodating a first unit is provided at one end thereof and a movement space connected to the accommodation portion is formed;
a first unit carrier configured to move in the movement space; and
a unit support portion provided in the accommodation portion,
wherein the unit support portion is pressed by one end of the first unit carrier as the first unit carrier moves in a direction becoming closer to the accommodation portion, and is moved from a restriction position in which at least one end thereof contacts the first unit accommodated in the accommodation portion to a release position in which at least one end thereof moves away from the first unit accommodated in the accommodation portion as the unit support portion is pressed by the first unit carrier.

2. The applicator of claim 1, wherein the first unit is supported by one end of the unit support portion positioned at the restriction position so that deviation from the accommodation portion is restricted, and the deviation restriction of the first unit by the unit support portion is released as the unit support portion moves to the release position.

3. The applicator of claim 1 or 2, wherein a second unit, intended to be delivered to the first unit, is detachably disposed at one end of the first unit carrier, and the first unit carrier is configured to move together with the second unit from an initial position, in which the second unit is spaced apart from the first unit, toward an insertion position, in which the second unit is delivered to the first unit.

4. The applicator of claim 3, wherein the unit support portion is pressed by the one end of the first unit carrier which has moved to the insertion position, thereby moving from the restriction position to the release position.

5. The applicator of claim 1 or 2, wherein the first unit carrier moves from an initial position spaced apart from the first unit to an insertion position in which the unit support portion is pressed.

6. The applicator of any one of the preceding claims, wherein the first unit carrier comprises:
a first unit carrier body, at least a portion of which is accommodated in the movement space and which is provided to move in the movement space;
an extension arm extending from a side end of the first unit carrier body and formed to extend toward the accommodation portion; and
an extension arm pushing portion formed to protrude from a leading end portion of the extension arm adjacent to the accommodation portion.

7. The applicator of claim 6, wherein the extension arm pushing portion comprises an inclined surface configured to assist the unit support portion in moving to a position away from the accommodation portion.

8. The applicator of any one of claims 1 to 7, wherein the unit support portion comprises:
a support hook disposed in a support hook accommodation space formed to penetrate one end of the body housing at one side of the accommodation portion, the support hook comprising a catching portion having a shape protruding toward the accommodation portion and a pushed portion formed to protrude in a direction opposite to the catching portion; and
a support hook connection portion extending from an end portion of the body housing forming the support hook accommodation space so as to be connected to the support hook and maintaining the support hook in the support hook accommodation space.

9. The applicator of claim 8, wherein the unit support portion is moved from the restriction position to the release position as the support hook connection portion is deformed.

10. The applicator of claim 8 or 9, wherein one end of the first unit is provided with a first unit housing groove formed to be recessed in a shape corresponding to the catching portion so that at least one end of the catching portion positioned at the restriction position is inserted and disposed.

11. The applicator of any one of claims 8 to 10, wherein the pushed portion is provided with a pushed portion inclined surface so that the pushed portion slidingly moves, as the pushed portion is pressed in contact with one end of the first unit carrier.

12. The applicator of any one of claims 6 to 11, wherein the body housing further comprises a column disposed in the body housing to partition the movement space, and a carrier slit, in which at least a portion of the extension arm is accommodated, is formed by incision in an end portion of the column along the first direction.

13. An applicator assembly, comprising:
a body housing in which an accommodation portion, capable of accommodating a transmission unit, is provided at one end thereof, and a movement space connected to the accommodation portion through an opening is formed therein;
a sensor unit carrier provided to move in the movement space, the sensor unit carrier having a sensor unit, intended to be delivered to the transmission unit, separably fixed at one end thereof; and
unit support portions disposed in pairs in a symmetrical shape on both side ends of the opening, the unit support portions having end portions in contact with the transmission unit accommodated in the accommodation portion to restrict deviation of the transmission unit from the accommodation portion,
wherein a spacing width between the paired unit support portions is wider after completion of the delivery of the sensor unit to the transmission unit than before the delivery of the sensor unit to the transmission unit.

14. The applicator assembly of claim 13, wherein the unit support portion is integrally formed with the body housing.

15. The applicator assembly of claim 13 or 14, wherein the deviation restriction of the transmission unit by the unit support portion is released as the sensor unit is delivered to the transmission unit.
